# EUROPÄISCHE PATENTSCHRIFT

(11) **EP 0 577 648 B1**
(45) Veröffentlichungstag und Bekanntmachung des Hinweises auf die Patenterteilung: **27.06.2001**
(21) Anmeldenummer: 92906855.9
(22) Anmeldetag: 24.03.1992
(51) Int. Cl.: A61K 47/48, C12N 15/87

(54) **NEUE PROTEIN-POLYKATION-KONJUGATE**
NOVEL PROTEIN POLYCATION CONJUGATES
NOUVEAUX CONJUGUES DE PROTEINES ET DE POLYCATIONS

(30) Priorität: 29.03.1991 DE 4110409
(43) Veröffentlichungstag der Anmeldung: 12.01.1994
(73) Patentinhaber: Boehringer Ingelheim International GmbH, 55218 Ingelheim am Rhein (DE); GENENTECH, INC., South San Francisco California 94080 (US)
(72) Erfinder: BIRNSTIEL, Max, L., A-1100 Wien (AT); COTTEN, Matthew, A-1130 Wien (AT); WAGNER, Ernst, A-2103 Langenzersdorf (AT)
(74) Vertreter: Laudien, Dieter, Dr.
(86) Internationale Anmeldenummer: EP9200642
(87) Internationale Veröffentlichungsnummer: WO9217210

(56) Entgegenhaltungen:
- EP-A- 0 388 758
- WO-A-88/05077
- WO-A-90/01951
- WO-A-91/04753
- WO-A-91/17773
- Cellular & Molecular Biology, Band 28, no. 1, 1982, (Oxford, GB), J.C. STAVRIDIS et al.: "Use of transferrin as a gene-carrier to the erythroid cells of the marrow", Seiten 15-18, siehe Seite 15, Zusammenfassung
- Proc. Natl. Acad. Sci. USA, Band 87, Mai 1990, (Washington, DC, US), E. WAGNER et al.: "Transferrin-polycation conjugates as carriers for DNA uptake into cells", Seiten 3410-3414, siehe Seite 3410, Zusammenfassung

## Beschreibung

Die Erfindung betrifft neue Protein-Polykation-Konjugate für den Transport von zu Polykationen affinen Verbindungen, insbesondere Nukleinsäuren, in menschliche oder tierische Zellen.

Nukleinsäuren als therapeutisch wirksame Substanzen haben in den letzten Jahren zunehmend an Bedeutung gewonnen.

Antisense RNAs und -DNAs haben sich als wirksame Mittel für die selektive Inhibierung bestimmter Gensequenzen erwiesen. Ihre Wirkungsweise ermöglicht ihre Anwendung als Therapeutika zur Blockierung der Expression bestimmter Gene (wie deregulierter Onkogene oder viraler Gene) in vivo. Es wurde bereits gezeigt, daß kurze Antisense-'Oligonukleotide in Zellen importiert werden und dort ihre inhibierende Wirkung ausüben können (Zamecnik et al., 1986), wenngleich ihre intrazelluläre Konzentration, u.a. wegen ihrer beschränkten Aufnahme durch die Zellmembran auf Grund der starken negativen Ladung der Nukleinsäuren, gering ist.

Ein weiterer Ansatz zur selektiven Inhibierung von Genen besteht in der Anwendung von Ribozymen. Auch hier besteht das Bedürfnis, eine möglichst hohe Konzentration von aktiven Ribozymen in der Zelle zu gewährleisten, wofür der Transport in die Zelle einer der limitierenden Faktoren ist.

Es wurden bereits mehrere Lösungen vorgeschlagen, den Transport von Nukleinsäuren in lebende Zellen, der bei deren therapeutischer Anwendung einen der limitierenden Faktoren darstellt, zu verbessern.

Einer der bekannten Lösungsansätze, inhibierende Nukleinsäure in die Zelle zu befördern, besteht in direkten Modifikationen der Nukleinsäuren, z.B. durch Substitution der geladenen Phosphodiestergruppen durch ungeladene Gruppen. Eine weitere Möglichkeit der direkten Modifikation besteht in der Verwendung von Nukleosidanalogen. Diese Vorschläge weisen jedoch verschiedene Nachteile auf, z.B. geringere Bindung an das Zielmolekül, geringere Hemmwirkung, mögliche Toxizität.

Bedarf an einem effizienten System für das Einführen von Nukleinsäure in lebende Zellen besteht außerdem vor allem im Rahmen der Gentherapie. Dabei werden Gene in Zellen eingeschleust, um in vivo die Synthese therapeutisch wirksamer Genprodukte zu erzielen, z.B. um im Falle eines genetischen Defekts das fehlende Gen zu ersetzen. Die "klassische" Gentherapie beruht auf dem Prinzip, durch eine einmalige Behandlung eine dauernde Heilung zu erzielen. Daneben besteht jedoch Bedarf an Behandlungsmethoden, bei denen die therapeutisch wirksame DNA (oder auch mRNA) wie ein Medikament ("Gentherapeutikum") je nach Bedarf einmalig oder wiederholt verabreicht wird. Beispiele für genetisch bedingte Erkrankungen, bei denen die Gentherapie einen erfolgversprechenden Ansatz darstellt, sind Hämophilie, beta-Thalassämie und "Severe Combined Immune Deficiency" (SCID), ein Syndrom, das durch einen genetisch bedingten Mangel des Enzyms Adenosindeaminase hervorgerufen wird. Anwendungsmöglichkeiten bestehen weiters bei der Immunregulation, wobei durch Verabreichung funktioneller Nukleinsäure, die für ein sekretiertes Protein-Antigen oder für ein nicht-sezerniertes Protein-Antigen kodiert, mittels einer Impfung eine humorale oder intrazelluläre Immunität erzielt wird. Weitere Beispiele für genetische Defekte, bei denen eine Verabreichung von Nukleinsäure, die für das defekte Gen kodiert, z.B. in individuell auf den Bedarf abgestimmter Form verabreicht werden kann, sind Muskeldystrophie (Dystrophin-Gen), Cystische Fibrose ("Cystic fibrosis transmembrane conductance regulator gene"), Hypercholesterolämie (LDL-Rezeptor-Gen). Gentherapeutische Behandlungsmethoden sind weiters potentiell dann von Bedeutung, wenn Hormone, Wachstumsfaktoren oder cytotoxisch oder immunmodulierend wirkende Proteine im Organismus synthetisiert werden sollen.

Die bisher am weitesten fortgeschrittenen Technologien für die Anwendung von Nukleinsäuren im Rahmen der Gentherapie benutzen retrovirale Systeme für den Transfer von Genen in die Zelle (Wilson et al., 1990, Kasid et al., 1990). Die Verwendung von Retroviren ist jedoch problematisch, weil sie, zumindest zu einem geringen Prozentsatz, die Gefahr von Nebenwirkungen wie Infektion mit dem Virus (durch Rekombination mit endogenen Viren und mögliche anschließende Mutation zur pathogenen Form) oder Entstehung von Krebs in sich birgt. Außerdem ist die stabile Transformation der somatischen Zellen des Patienten, wie sie mit Hilfe von Retroviren erzielt wird, nicht in jedem Fall wünschenswert, weil dadurch die Behandlung, z.B. bei Auftreten von Nebeneffekten, nur mehr schwierig rückgängig gemacht werden kann.

Es wurde daher nach alternativen Methoden gesucht, um die Expression von nicht-replizierender DNA in der Zelle zu ermöglichen.

Für die Gentransformation von Säugetierzellen in vitro sind verschiedene Techniken bekannt, deren Anwendbarkeit in vivo jedoch beschränkt ist (dazu zählen das Einbringen von DNA mittels Liposomen, Elektroporation, Mikroinjektion, Zellfusion, DEAE-Dextran oder die Calciumphosphat-Präzipitationsmethode).

Neuere Bestrebungen, Methoden für den in vivo Gentransfer zu entwickeln, konzentrieren sich auf die Verwendung des kationischen Lipidreagens Lipofektin, von dem gezeigt werden konnte, daß ein mit Hilfe dieses Reagens injiziertes Plasmid im Organismus exprimiert wird (Nabel et al., 1990).

Eine weitere kürzlich entwickelte Methode benutzt Mikropartikel aus Wolfram oder Gold, an denen DNA absorbiert ist, mit denen die Zelle mit hoher Energie beschossen werden (Johnston, 1990, Yang et al., 1990). Expression der DNA konnte in verschiedenen Geweben nachgewiesen werden.

Ein in vivo anwendbares lösliches System, das DNA zielgerichtet in die Zellen befördert, wurde für Hepatozyten entwickelt und beruht auf dem Prinzip, Polylysin an ein Glykoprotein, auf das ein an der Hepatozytenoberfläche vorhandener Rezeptor anspricht, zu koppeln und daraufhin durch Zugabe von DNA einen löslichen Glykoprotein/Polylysin/DNA-Komplex zu bilden, der in die Zelle aufgenommen wird und nach Aufnahme des Komplexes in die Zelle die Expression der DNA-Sequenz ermöglicht (Wu und Wu, 1987).

Dieses System ist spezifisch für Hepatozyten und wird hinsichtlich seiner Funktion durch den relativ gut charakterisierten Aufnahmemechanismus durch den Asiologlykoproteinrezeptor definiert.

Ein breiter anwendbares, effizientes Transportsystem benutzt den Transferrinrezeptor für die Aufnahme von Nukleinsäuren in die Zelle mittels Transferrrin-Polykation-Konjugaten. Dieses System ist Gegenstand der Europäischen Patentanmeldung EP A1 388.758. Es konnte gezeigt werden, daß Transferrin-Polykation/DNA-Komplexe effizient in lebende Zellen aufgenommen und internalisiert werden, wobei als Polykationanteil der Komplexe Polylysin verschiedenen Polymerisationsgrades und Protamin verwendet wurden. Mit Hilfe dieses Systems wurde u.a. ein das erbB-Onkogen inhibierendes Ribozymgen in erbB-transformierte Hühnerzellen eingebracht und die erbB inhibierende Wirkung nachgewiesen.

Der vorliegenden Erfindung lag die Aufgabe zugrunde, ein System bereitzustellen, mit Hilfe dessen ein selektiver Transport von Nukleinsäuren in höhere eukaryotische Zellen ermöglicht wird.

Es wurde überraschend festgestellt, daß Antikörper, die an ein Zelloberflächenprotein binden, für den Transport von Nukleinsäuren in höhere eukaryotische Zellen herangezogen werden können, wenn sie mit Polykationen konjugiert werden.

Es konnte in Vorversuchen gezeigt werden, daß das vom HIV-Virus bei der Infektion benutzte Zelloberflächenprotein CD4, für den Transport von Nukleinsäure in die Zelle ausgenutzt werden kann, indem die zu importierende Nukleinsäure mit einem Protein-Polykation-Konjugat komplexiert wird, dessen Proteinanteil ein gegen CD4 gerichteter Antikörper ist, und CD4 exprimierende Zellen mit den erhaltenen Protein-Polykation/DNA-Komplexen in Berührung gebracht werden.

Im Rahmen der vorliegenden Erfindung konnte in Vorversuchen weiters gezeigt werden, daß mit Hilfe von Antikörper-Polykation-Konjugaten, enthaltend einen Antikörper gegen CD7, DNA in Zellen der T-Zell-Abstammungslinie ("T-cell lineage") eingeführt und in diesen Zellen exprimiert wird. (CD7 ist ein Zelloberflächenpotein mit derzeit noch unbekannter physiologischer Rolle, das auf Thymocyten und reifen T-Zellen nachgewiesen wurde. CD7 ist ein verläßlicher Marker für die akute T-Zellenleukämie (Aruffo und Seed, 1987)).

Für Antikörper-Polykation-Konjugate, die einen gegen die Membranfraktion von Rattenpankreaskarzinom-Zellen gerichteten Antikörper enthielten, konnte ebenfalls nachgewiesen werden, daß DNA, die mit den Konjugaten komplexiert wird, in solche Zellen eingeführt und in ihnen exprimiert wird.

Im Rahmen der vorliegenden Erfindung konnte somit anhand von Antikörper-Polykation-Konjugaten mit verschiedenen Antikörper-Anteilen gezeigt werden, daß mit Hilfe solcher Konjugate in Zellen, die das jeweilige Oberflächenantigen exprimieren, gegen das der Antikörper gerichtet ist, Internalisierung und Expression von DNA erzielt werden kann.

Die Erfindung betrifft somit neue Protein-Polykation-Konjugate, die befähigt sind, mit Nukleinsäuren Komplexe zu bilden, wobei der Proteinanteil ein Antikörper gegen ein Zelloberflächenprotein ist, der die Fähigkeit besitzt, an das Zelloberflächenprotein zu binden, so daß die gebildeten Komplexe in menschliche oder tierische Zellen, das Zelloberflächenprotein exprimieren, aufgenommen werden, ausgenommen Antikörper, gerichtet gegen ein T-Zell-Oberflächenprotein.

Im folgenden werden Antikörper gegen Zelloberflächenproteine der Zielzellen als "Antikörper" bezeichnet.

Gegenstand der Erfindung sind weiters Antikörper-Polykation/Nukleinsäure-Komplexe, in denen die erfindungsgemäßen Konjugate mit einer in die Zielzellen, die das Zelloberflächenantigen exprimieren, gegen das der Antikörper gerichtet ist, zu transportierenden Nukleinsäure komplexiert ist.

Im Rahmen der vorliegenden Erfindung konnte gezeigt werden, daß DNA als Bestandteil der erfindungsgemäßen Komplexe effizient in Zellen, die das jeweilige Antigen exprimieren, gegen die die Antikörper gerichtet sind, aufgenommen und exprimiert wird, wobei die Aufnahme von DNA in die Zelle mit steigendem Konjugatgehalt zunahm.

Als Antikörper kommen alle diejenigen, insbesondere monoklonalen Antikörper gegen Zelloberflächen-Antigene bzw. die Fragmente davon in Betracht, die an an das Zelloberflächen-Antigen binden, z.B. Fab'-Fragmente (Pelchen-Matthews et al., 1989).

Anstelle konventioneller monoklonaler Antikörper bzw. deren Fragmente können Antikörpervarianten bzw. -abschnitte, bestehend aus einer Kombination von Segmenten der schweren und leichten Kette oder gegebenenfalls der schweren Kette allein, verwendet werden. Die Herstellung solcher Antikörper mit Hilfe gentechnischer Methoden, z.B. durch Klonierung mittels Polymerase-Kettenreaktion und Expression in E.coli, wurde kürzlich beschrieben (z.B. Sastry et al., 1989; Orlandi et al., 1989; Chaudhary et al., 1990). Um bei therapeutischen Anwendungen beim Menschen, insbesondere bei Behandlungen in vivo über einen längeren Zeitraum, Immunantworten zu vermeiden, werden für solche Anwendungen humanisierte (z.B. Co und Queen, 1991) oder humane Antikörper bevorzugt. Eine Übersicht über monoklonale Antikörper und gentechnisch hergestellte Antikörpervarianten wird von Waldmann, 1991, gegeben. Antikörper gegen Oberflächenmoleküle auf humanen Leukozyten sind von Knapp et al., 1989, angeführt.

Die Wahl des Antikörpers wird vor allem durch die Zielzellen bestimmt, z.B. durch bestimmte Oberflächenantigene oder Rezeptoren, die für einen Zelltyp spezifisch bzw. weitgehend sind und somit einen gerichteten Import der Nukleinsäure in diesen Zelltyp ermöglichen.

Die erfindungsgemäßen Konjugate ermöglichen je nach Oberflächenantigen, gegen das der im Konjugat enthaltene Antikörper gerichtet ist, eine engere oder breitere Selektivität hinsichtlich der mit Nukleinsäure zu behandelnden Zellen und somit einen flexiblen Einsatz therapeutisch oder gentherapeutisch wirksamer Nukleinsäure.

Im Rahmen der vorliegenden Erfindung sind als Konjugat-Komponente Antikörper oder Fragmente davon geeignet, die an die Zelle binden, wodurch die Konjugat/DNA-Komplexe, insbesondere über Endozytose, internalisiert werden, oder Antikörper(fragmente), deren Bindung/Internalisierung über Fusion mit Zellmembranelementen erfolgt.

Wesentlich für die Eignung von Antikörper(fragmente)n im Rahmen der vorliegenden Erfindung ist,
a) daß sie vom spezifischen Zelltyp, in den die Nukleinsäure eingebracht werden soll, erkannt werden und ihre Bindungs- bzw. Internalisierungsfähigkeit nicht oder nicht wesentlich beeinträchtigt wird, wenn sie mit dem Polykation konjugiert werden, und
b) daß sie im Rahmen dieser Eigenschaft in der Lage sind, Nukleinsäure auf dem von ihnen benutzten Weg in die Zelle "huckepack" mitzunehmen.

Unter der Voraussetzung, daß sie die unter a) und b) definierten Bedingungen erfüllen, sind grundsätzlich alle gegen Oberflächenantigene gerichteten Antikörper für die Anwendung im Rahmen der vorliegenden Erfindung geeignet. Dazu zählen Antikörper gegen Zelloberflächenproteine, die spezifisch auf einem bestimmten Zelltypus exprimiert werden.

Zu im Rahmen der vorliegenden Erfindung geeigneten Antikörpern zählen Antikörper gegen Rezeptoren, die im Rahmen der vorliegenden Erfindung unter die Definition "Zelloberflächenproteine" fallen. Beispiele für Rezeptoren sind der Transferrin-Rezeptor, der Hepatozyten-Asialoglykoproteinrezeptor, Rezeptoren für Hormone oder Wachstumsfaktoren (Insulin-, EGF-Rezeptor), Rezeptoren für cytotoxisch wirksame Substanzen wie TNF oder Rezeptoren, die die extrazelluläre Matrix binden, wie der Fibronectin-Rezeptor oder der Vitronectin-Rezeptor. Geeignet sind auch Antikörper gegen Liganden für Zelloberflächenrezeptoren, soweit sie die Bindungsfähigkeit des Liganden an seinen Rezeptor nicht beeinträchtigen.

Für die gezielte Anwendung auf Tumorzellen kommen vor allem Antikörper gegen spezifisch auf den jeweiligen Tumorzellen exprimierte Zelloberflächenproteine, sog. Tumormarker, in Betracht.

Zu im Rahmen der vorliegenden Erfindung geeigneten Polykationen zählen homologe Polykationen wie Polylysin, Polyarginin, Polyornithin oder heterologe Polykationen mit zwei oder mehr unterschiedlichen positiv geladenen Aminosäuren zu verstehen, wobei diese Polykationen verschiedene Kettenlänge aufweisen können, weiters nichtpeptidische synthetische Polykationen wie Polyethylenimin. Geeignet sind weiters naürliche DNA bindende Proteine polykationischen Charakters wie Histone oder Protamine bzw. Analoge oder Fragmente davon.

Die Größe der Polykationen ist nicht kritisch; im Falle von Polylysin wird sie z.B. bevorzugt so gewählt, daß die Summe der positiven Ladungen etwa 20 bis 1000 beträgt, sie wird auf die zu transportierende Nukleinsäure abgestimmt. Für eine vorgegebene Nukleinsäurelänge ist die Länge des Polykations nicht kritisch. Hat z.B. die DNA 6.000 bp und 12.000 negative Ladungen, beträgt die Menge an Polykation z.B. 60 Mol Polylysin 200 oder 30 Mol Polylysin 400 oder 120 Mol Polylysin 100, etc. Der Durchschnittsfachmann ist in der Lage, mit Hilfe einfach durchzuführender Routineexperimente auch andere Kombinationen von Polykationgrößen und molaren Mengen zu wählen.

Die erfindungsgemäßen Antikörper-Polykation-Konjugate werden bevorzugt auf chemischem Weg in für die Kopplung von Peptiden an sich bekannter Weise hergestellt, wobei, falls erforderlich, die Einzelkomponenten vor der Kopplungsreaktion mit Linkersubstanzen versehen werden (diese Maßnahme ist dann erforderlich, wenn von vornherein keine für die Kopplung geeignete funktionelle Gruppe, z.B. eine Mercapto- oder Alkoholgruppe, verfügbar ist. Bei den Linkersubstanzen handelt es sich um bifunktionelle Verbindungen, die zunächst mit funktionellen Gruppen der Einzelkomponenten zur Reaktion gebracht werden, worauf die Kopplung der modifizierten Einzelkomponenten durchgeführt wird.

Je nach gewünschten Eigenschaften der Konjugate, insbesondere im Hinblick auf deren Stabilität, kann die Kopplung erfolgen über
a) Disulfidbrücken, die unter reduzierenden Bedingungen wieder gespalten werden können (z.B. bei Verwendung von Succinimidylpyridyldithiopropionat (Jung et al., 1981).
b) Unter biologischen Bedingungen weitgehend stabile Verbindungen (z.B. Thioether durch Reaktion von Maleimido-Linkern mit Sulfhydrylgruppen des an die zweite Komponente gebundenen Linkers).
c) Unter biologischen Bedingungen labile Brücken, z.B. Esterbindungen, oder unter schwach sauren Bedingungen instabile Acetal- oder Ketalbindungen.

Im Fall der Verwendung der oben angeführten gentechnisch hergestellten Antikörper ist es auch möglich, die erfindungsgemäßen Konjugate auf rekombinantem Weg herzustellen, was den Vorteil hat, genau definierte, einheitliche Verbindungen erhalten zu können, während bei der chemischen Kopplung zunächst Konjugat-Gemische entstehen, die aufgetrennt werden müssen.

Die rekombinante Herstellung der erfindungsgemäßen Konjugate ist nach für die Herstellung von chimären Polypeptiden bekannten Methoden durchführbar. Dabei können die polykationischen Peptide hinsichtlich ihrer Größe und Aminosäuresequenz variiert werden. Die gentechnologische Herstellung bietet auch den Vorteil, den Antikörper-Anteil des Konjugats modifizieren zu können, indem z.B. durch geeignete Mutationen die Bindungsfähigkeit an das Zelloberflächenprotein gesteigert werden kann oder ein Antikörper-Anteil, verkürzt auf den für die Bindung an das Zelloberflächenprotein maßgeblichen Teil des Moleküls, verwendet wird. Besonders zweckmäßig für die rekombinante Herstellung der erfindungsgemäßen Konjugate ist die Verwendung eines Vektors, der die für den Antikörper-Anteil kodierende Sequenz sowie einen Polylinker enthält, in den die jeweils benötigte für das polykationische Peptid kodierende Sequenz eingesetzt wird. Auf diese Weise kann ein Satz von Expressionsplasmiden erhalten werden, von dem bei Bedarf das die gewünschte Sequenz enthaltende Plasmid zur Expression des erfindungsgemäßen Konjugats herangezogen wird.

Falls der Antikörper geeignete Kohlenhydratketten aufweist, kann er mit dem Polykation über eine oder mehrere dieser Kohlenhydratketten verbunden werden. Solche Konjugate haben gegenüber zu den mittels herkömmlicher Kopplungsverfahren hergestellten Konjugaten den Vorteil, daß sie frei von Modifikationen sind, die von den verwendeten Linkersubstanzen stammen. Eine geeignete Methode zur Herstellung von Glykoprotein-Polykation-Konjugaten ist in der deutschen Patentanmeldung P 41 15 038.4 geoffenbart; sie wurde kürzlich von Wagner et al., 1991, beschrieben.

Das molare Verhältnis Antikörper:Polykation beträgt bevorzugt 10:1 bis 1:10, wobei zu berücksichtigen ist, daß es zur Ausbildung von Aggregaten kommen kann. Dieses Verhältnis kann jedoch bei Bedarf innerhalb weiterer Grenzen liegen, solange die Bedingung erfüllt ist, daß Komplexierung der Nukleinsäure(n) stattfindet und gewährleistet ist, daß der gebildete Komplex an das Zelloberflächenprotein gebunden und in die Zelle befördert wird. Dies kann durch einfach durchzuführende Versuche von Fall zu Fall überprüft werden, z.B indem Zellinien, die das Zelloberflächenantigen exprimieren, mit den erfindungsgemäßen Konjugaten in Berührung gebracht werden und daraufhin auf das Vorhandensein von Nukleinsäure in der Zelle untersucht werden, z.B. durch Southern Blot Analyse, Hybridisierung mit radioaktiv markierten komplementären Nukleinsäuremolekülen, durch Amplifikation mit Hilfe der PCR oder durch Nachweis des Genproduktes eines Reportergens.

Für bestimmte Anwendungsfälle, vor allem für das Screening zum Auffinden geeigneter Antikörper, kann es vorteilhaft sein, den Antikörper nicht direkt an das Polykation zu koppeln: für eine effiziente chemische Kopplung ist im allgemeinen eine größere Menge (mehr als 1 mg) Ausgangsantikörper erforderlich, außerdem wird durch die Kopplung gegebenenfalls die Antikörper-Bindungsdomäne inaktiviert. Um dieses Probleme zu umgehen und ein rasches Screening geeigneter Antikörper zu ermöglichen, kann zunächst ein Protein A-Polykation-Konjugat hergestellt werden, an das anschließend, gegebenenfalls in bereits mit Nukleinsäure komplexierter Form, unmittelbar vor der Transfektion der Zellen, der Antikörper über die Fc-Bindungsdomäne von Protein A gebunden wird (Surolia et al., 1982). Die mit den Protein A-Konjugaten gebildeten Nukleinsäure-Komplexe ermöglichen ein rasches Testen von Antikörpern auf ihre Eignung für den Import von Nukleinsäure in den jeweiligen zu behandelnden Zelltyp. Die Kopplung von Protein A mit dem jeweiligen Polykation erfolgt in analoger Weise wie die direkte Kopplung mit dem Antikörper. Bei der Anwendung von Protein A-Antikörper-Polykation-Konjugaten kann es vorteilhaft sein, die zu behandelnden Zellen zuerst mit dem Antikörper zu inkubieren, die Zellen von überschüssigem Antikörper zu befreien und anschließend mit dem Protein A-Polykation/Nukleinsäure-Komplex zu behandeln. Gegebenenfalls ist Protein A modifiziert, z.B. durch Anteile von Protein G, um seine Affinität zu den Antikörpern zu verstärken.

Bei den in die Zelle zu transportierenden Nukleinsäuren kann es sich um DNAs oder RNAs handeln, wobei hinsichtlich der Nukleotidsequenz keine Beschränkungen bestehen. Die Nukleinsäuren können modifiziert sein, sofern die Modifikation den polyanionischen Charakter der Nukleinsäuren nicht beeinträchtigt; zu diesen Modifikationen zählen z.B. die Substitution der Phosphodiestergruppe durch Phosphorothioate oder in der Verwendung von Nukleosidanalogen. Derartige Modifikationen sind dem Fachmann geläufig, eine Übersicht über Vertreter modifizierter Nukleinsäuren, die im allgemeinen als Nukleinsäureanaloga bezeichnet werden, sowie deren Wirkprinzip, sind in der Übersicht von Zon (1988) angegeben.

Bezüglich der Größe der Nukleinsäuren ermöglicht die Erfindung ebenfalls Anwendungen in einem weiten Bereich. Hinsichtlich der oberen Grenze besteht keine durch die erfindungsgemäßen Konjugate bedingte prinzipielle Limitierung, solange gewährleitstet ist, daß die Antikörper-Polykation/Nukleinsäurekomplexe in die Zelle befördert werden. Eine etwaige Begrenzung nach unten ergibt sich aus anwendungsspezifischen Gründen, weil z.B. Antisense-Oligonukleotide unter etwa 10 Nukleotiden auf Grund zu geringer Spezifität kaum für die Anwendung in Frage kommen. Mit Hilfe der erfindungsgemäßen Konjugate können auch Plasmide in die Zelle befördert werden. Kleinere Nukleinsäuren, z.B. für Antisense-Anwendungen, gegebenenfalls in Tandem, können auch als integrale Bestandteile von größeren Genkonstrukten, von denen sie in der Zelle transkribiert werden, eingesetzt werden.

Es ist auch möglich, gleichzeitig verschiedene Nukleinsäuren mit Hilfe der erfindungsgemäßen Konjugate in die Zelle zu befördern.

Beispiele für geeignete Nukleinsäuren mit inhibierender Wirkung sind die bereits angeführten Antisenseoligonukleotide oder Ribozyme mit Komplementarität zu für die Virusreplikation essentiellen Genabschnitten.

Bevorzugt als Nukleinsäureanteil der erfindungsgemäßen Antikörper-Polykation-Nukleinsäurekomplexe mit inhibierender Wirkung aufgrund von Komplementarität ist Antisense-DNA, Antisense-RNA oder ein Ribozym bzw. das dafür kodierende Gen. Bei der Anwendung von Ribozymen und Antisense-RNAs ist es besonders vorteilhaft, die dafür kodierenden Gene, gegebenenfalls zusammen mit einem Carriergen, einzusetzen. Durch die Einführung des Gens in die Zelle wird gegenüber dem Import der RNA als solcher eine beträchtliche Amplifikation der RNA und damit ein für die angestrebte Hemmung der biologischen Reaktion ausreichender Vorrat gewährleistet. Besonders geeignete Carriergene sind die für die Transkription durch Polymerase III erforderlichen Transkriptionseinheiten, z.B. tRNA-Gene. In diese können z.B. Ribozymgene derart eingefügt werden, daß bei Transkription das Ribozym Teil eines kompakten Polymerase III-Transkripts ist. Geeignete genetische Einheiten, enthaltend ein Ribozymgen und ein von Polymerase III transkribiertes Carriergen, sind in der Europäischen Patentanmeldung EP A1 0 387 775 geoffenbart. Mit Hilfe des Transportsystems gemäß der vorliegenden Erfindung kann die Wirkung dieser genetischen Einheiten verstärkt werden, indem eine erhöhte Ausgangskonzentration des Gens in der Zelle gewährleistet wird.

Als Zielsequenzen für die Konstruktion komplementärer Antisenseoligonukleotide oder Ribozyme bzw. der dafür kodierenden Gene, die bei der Therapie von AIDS zur Anwendung kommen können, sind grundsätzlich sämtliche Sequenzen des HIV-Gens geeignet, deren Blockierung die Inhibierung der viralen Replikation und Expression zur Folge hat. In erster Linie in Frage kommende Zielsequenzen sind Sequenzen mit regulatorischer Funktion, vor allem des tat-, rev- oder nef-Gens. Geeignete Sequenzen sind weiters die Initiations-, Polyadenylierungs-, Splicing tRNA Primer-Bindungsstelle (PBS) der LTR-Sequenz oder die tar-Sequenz.

Außer Nukleinsäuremolekülen, die aufgrund ihrer Komplementarität zu viralen Genen inhibieren, können auch Gene mit anderem Wirkmechanismus eingesetzt werden, z.B. solche, die für Virusproteine, enthaltend sog. trans-dominante Mutationen, kodieren (Herskowitz, 1987). Die Expression der Genprodukte in der Zelle resultiert in Proteinen, die in ihrer Funktion das entsprechenden Wildtyp-Virusprotein dominieren, womit dieses seine für die Virusreplikation normalerweise geleistete Aufgabe nicht erfüllen kann und die Virusreplikation wirksam inhibiert wird. Geeignet sind grundsätzlich trans-dominante Mutanten von Virusproteinen, die für die Replikation und Expression erforderlich sind, z.B. Gag-, Tat- und Rev-Mutanten, von denen inhibierende Wirkung auf die HIV-Replikation nachgewiesen wurde (Trono et al., 1989; Green et al., 1989; Malim et al., 1989).

Vertreter therapeutisch wirksamer Nukleinsäuren sind weiters solche mit inhibierender Wirkung auf Onkogene.

Mit Hilfe der vorliegenden Erfindung können auch Gene bzw. Abschnitte davon in die Zelle transportiert werden, deren Expressionsprodukte eine Funktion bei der Signalübertragung haben, um auf diese Weise die Signalübertragung in den Zielzellen positiv zu beeinflussen und damit die Überlebensfähigkeit der Zielzellen zu steigern.

Prinzipiell sind sämtliche Gene bzw. Genabschnitte im Rahmen der vorliegenden Erfindung geeignet, die in in den das Zelloberflächenprotein exprimierenden Zellen einen therapeutischen oder gentherapeutischen Effekt haben.

Beispiele für Gene, die in der Gentherapie eingesetzt und in die Zelle mit Hilfe der vorliegenden Erfindung eingebracht werden können, sind Faktor VIII (z.B. Wood et al., 1984), Faktor IX (eingesetzt bei Hämophilie; z.B. Kurachi und Davie, 1982), Adenosindeaminase (SCID; z.B. Valerio et al., 1984), α-1-Antitrypsin (Lungenenphysem; z.B. Ciliberto et al., 1985) oder das "cystic fibrosis transmembrane conductance regulator gene" (Riordan et al., 1989). Diese Beispiele stellen keinerlei Beschränkung dar.

Das Verhältnis Nukleinsäure:Konjugat kann innerhalb eines weiten Bereichs schwanken, wobei es nicht unbedingt erforderlich sein muß, sämtliche Ladungen der Nukleinsäure zu neutralisieren. Dieses Verhältnis wird von Fall zu Fall nach Kriterien wie Größe und Struktur der zu transportierenden Nukleinsäure, Größe des Polykations, Anzahl und Verteilung seiner Ladungen, derart einzustellen sein, daß ein für die jeweilige Anwendung günstiges Verhältnis zwischen Transportfähigkeit und biologischer Aktivität der Nukleinsäure besteht. Dieses Verhältnis kann zunächst grob eingestellt werden, etwa an Hand der Verzögerung der Wanderungsgeschwindigkeit der DNA in einem Gel (z.B. mittels "Mobility Shift" auf einem Agarosegel) oder durch Dichtegradientenzentrifugation. Nach Erhalt dieses vorläufigen Verhältnisses kann es zweckmäßig sein, im Hinblick auf die in der Zelle maximal verfügbare Aktivität der Nukleinsäure Transportversuche mit dem radioaktiv markierten Komplex durchzuführen und den Konjugat-Anteil gegebenenfalls derart zu reduzieren, daß die verbleibenden negativen Ladungen der Nukleinsäure dem Transport in die Zelle nicht hinderlich sind.

Die Herstellung der Antikörper-Polykation/Nukleinsäure-Komplexe kann nach in an sich für die Komplexierung polyionischer Verbindungen bekannten Methoden vorgenommen wurden. Eine Möglichkeit, unkontrollierte Aggregation bzw. Ausfällung zu vermeiden, besteht darin, die beiden Komponenten bei hoher Verdünnung (≤100 µg) zu mischen.

Die über Endozytose in höhere eukaryotische Zellen aufnehmbaren Antikörper-Polykation-Nukleinsäure-Komplexe können zusätzlich eine oder mehrere Polykationen in nicht-kovalent gebundener Form, die gegebenenfalls identisch sind mit dem Polykation im Konjugat, derart enthalten, daß die durch das Konjugat erzielte Internalisierung und/oder Expression der Nukleinsäure gesteigert wird.

Mit Hilfe derartiger Maßnahmen, die Gegenstand der WO92/13570 sind, wird bei zumindest gleicher Transfektions/Expressions-Effizienz eine geringere Menge, bezogen auf die in die Zelle zu importierende Nukleinsäure-Menge, Antikörper-Polykation-Konjugat benötigt, was einerseits einen geringeren Synthese-Aufwand bedeutet. Eine geringere Konjugatmenge kann andererseits dann von Vorteil sein, wenn der Effekt vermieden werden soll, daß durch eine größere Anzahl von Antikörper-Molekülen innerhalb eines Komplexes gleich mehrere benachbarte "Andock-Stellen" besetzt werden und in der Folge für weitere Komplexe nicht mehr verfügbar sein können. Die Anzahl der in den Komplexen enthaltenen Menge an Antikörper auf das erforderliche Minimum zu beschränken, d.h. die Konjugatmenge möglichst gering zu halten und mit freiem Polykation zu verdünnen, ist insbesondere auch dann von vorteil, wenn die Zahl von Zelloberflächenproteinen auf den zu behandelnden Zielzellen gering ist.

Mit Hilfe solcher Maßnahmen kann die Leistungsfähigkeit von Konjugaten, die für sich weniger effizient sind, beträchtlich erhöht werden und die Leistungsfähigkeit von an sich schon sehr effizienten Konjugaten noch weiter gesteigert werden.

Hinsichtlich der qualitativen Zusammensetzung der erfindungsgemäßenm Komplexe werden im allgemeinen zunächst die in die Zelle zu importierende Nukleinsäure und der Antikörper festgelegt. Die Nukleinsäure wird vor allem durch den in der Zelle zu erzielenden biologischen Effekt definiert, z.B. durch die Zielsequenz des zu inhibierenden oder - im Falle der Anwendung im Rahmen der Gentherapie - des zur Expression zu bringenden Gens bzw. Genabschnitts, z.B. zwecks Substitution eines defekten Gens. Gegebenenfalls ist die Nukleinsäure modifiziert, bedingt z.B. durch eine für den jeweiligen Anwendungsfall erforderliche Stabilität.
Ausgehend von der Festlegung von Nukleinsäure und Antikörper wird das Polykation auf diese Parameter abgestimmt, wobei die Größe der Nukleinsäure, vor allem im Hinblick auf eine weitgehende Neutralisierung der negativen Ladungen, eine wesentliche Rolle spielt.

Für die Wahl der gegebenenfalls in den Komplexen enthaltenen nicht-kovalent gebundenen Polykationen ist entscheidend, daß durch ihren Zusatz gegenüber der durch die Konjugate erzielbaren Internalisierung/Expression der Nukleinsäure eine Steigerung bewirkt wird.

Ebenso wie die qualitative Zusammensetzung richtet sich auch die quantitative Zusammensetzung der Komplexe nach mehreren Kriterien, die miteinander in funktionellem Zusammenhang stehen, z.B. ob und in welchem Ausmaß es erforderlich oder erwünscht ist, die Nukleinsäure zu kondensieren, welche Ladung der Gesamtkomplex aufweisen soll, in welchem Ausmaß Bindungs- und Internalisierungsfähigkeit für den jeweiligen Zelltyp gegeben ist und in welchem Ausmaß deren Steigerung erwünscht bzw. erforderlich ist. Weitere Parameter für die Zusammensetzung des Komplexes sind die Zugänglichkeit des Antikörpers für das Zelloberflächenprotein, wobei dafür die Art maßgeblich ist, wie der Antikörper innerhalb des Komplexes der Zelle gegenüber präsentiert wird. Wesentlich ist weiters die Zugänglichkeit der Nukleinsäure in der Zelle, um deren bestimmungsgemäße Funktion auszuüben.

Die in nicht kovalent-gebundener Form in den Komplexen gegebenenfalls enthaltenen Polykationen können gleich oder verschieden sein von den im Konjugat enthaltenen. Ein wesentliches Kriterium für deren Auswahl ist die Größe der Nukleinsäure, insbesondere im Hinblick auf deren Kondensierung; bei kleineren Nukleinsäure-Molekülen ist im allgemeinen eine Kompaktierung nicht erforderlich. Die Auswahl der Polykationen nach Art und Menge wird außerdem auf das Konjugat abgestimmt, wobei vor allem das im Konjugat enthaltene Polykation zu berücksichtigen ist: ist das Polykation z.B eine Substanz, die keine oder nur geringe Fähigkeit zur DNA-Kondensation aufweist, ist es im Hinblick auf eine effiziente Internalisierung der Komplexe im allgemeinen zweckmäßig, solche Polykationen einzusetzen, die diese Eigenschaft in stärker ausgeprägtem Maß besitzen. Ist das im Konjugat enthaltene Polykation selbst eine Nukleinsäure kondensierende Substanz und wird bereits eine im Hinblick auf effiziente Internalisierung ausreichende Kompaktierung der Nukleinsäure bewirkt, wird zweckmäßigerweise ein Polykation verwendet, das aufgrund anderer Mechanismen eine Steigerung der Expression bewirkt.

Wesentlich für das gegebenenfalls im Komplex zusätzlich enthaltene nicht-kovalent gebundene Polykation ist seine Fähigkeit, Nukleinsäure zu kondensieren und/oder diese vor unerwünschtem Abbau in der Zelle zu schützen. Gegenstand der Erfindung ist weiters ein Verfahren zum Einführen von Nukleinsäure(n) in menschliche oder tierische Zellen, wobei bevorzugt ein unter physiologischen Bedingungen löslicher Antikörper-Polykation/Nukleinsäure-Komplex mit den Zellen in Berührung gebracht wird.

Im Rahmen der vorliegenden Erfindung wurde als DNA-Komponente das Luciferase-Gen als Reporter-Gen verwendet. (Aufgrund von in Vorversuchen erhaltenen Ergebnissen mit Transferrin-Polykation/DNA-Komplexen, in denen das Luciferase-Gen als Reportergen verwendet wurde, war gezeigt worden, daß aus der Effizienz des Imports des Luciferase-Gens auf die Anwendbarekeit anderer Nukleinsäuren geschlossen werden kann und die verwendete Nukleinsäure in qualitativer Hinsicht kein limitierender Faktor für die Anwendung von Protein-Polykation-DNA-Komplexen ist.)

Es kann für bestimmte Ausführungsformen der vorliegenden Erfindung vorteilhaft sein, Bedingungen zu schaffen, unter denen der Abbau der in die Zelle importierten Nukleinsäure verringert oder ausgeschaltet wird.

Bedingungen, unter denen der Abbau von Nukleinsäuren inhibiert wird, können durch Zusatz sog. lysosomatroper Substanzen geschaffen werden. Von diesen Substanzen ist bekannt, daß sie die Aktivität von Proteasen und Nukleasen in Lysosomen hemmen und somit den Abbau von Nukleinsäuren verhindern können (Luthman und Magnusson, 1983).

Zu diesen Substanzen zählen Chloroquin, Monensin, Nigericin, Ammoniumchlorid und Methylamin.

Das Erfordernis für die Anwendung einer Substanz aus der Gruppe der lysosomatropen Substanzen im Rahmen der vorliegenden Erfindung richtet sich u.a. nach dem zu behandelnden Zelltyp oder bei Verwendung unterschiedlicher Antikörper gegebenenfalls nach unterschiedlichen Mechanismen, über die die Aufnahme der Komplexe in die Zelle erfolgt. So wurde z.B. im Rahmen der vorliegenden Erfindung eine unterschiedliche Beeinflussung des DNA-Imports in die Zelle durch Chloroquin bei Verwendung unterschiedlicher Antikörper (monoklonale Anti-CD4-Antikörper) festgestellt.

Es ist in jedem Fall notwendig, das Erfordernis bzw. die Eignung derartiger Substanzen im Rahmen der vorliegenden Erfindung in Vorversuchen zu testen.

Gegenstand der Erfindung sind weiters pharmazeutische Zubereitungen, enthaltend als wirksame Komponente eine oder mehrere therapeutisch oder gentherapeutisch wirksame Nukleinsäure(n), komplexiert mit einem Antikörper-Polykation-Konjugat (Antikörper-Polykation-Konjugat und Nukleinsäure können auch getrennt vorliegen und unmittelbar vor der therapeutischen Anwendung komplexiert werden). Jeder pharmazeutisch annehmbare Träger, z.B. Kochsalzlösung, phosphatgepufferte Kochsalzlösung, oder andere Träger, in dem die erfindungsgemäßen Zusammensetzungen die geeigneten Löslichkeitseigenschaften aufweisen, können verwendet werden. Zur Formulierung wird auf Remington's Pharmaceutical Sciences, 1980, verwiesen.

Als therapeutisch wirksame Nukleinsäuren kommen die bereits angeführten Antisenseoligonukleotide oder Ribozyme bzw. die dafür kodierenden Gene oder für trans-dominante Mutanten kodierende Gene in Betracht, die inhibierende Wirkung auf in in den jeweiligen Zielzellen enthaltene endogene oder exogene Gene oder Genprodukte haben. Darunter sind z.B. diejenigen Gene zu verstehen, die aufgrund ihrer Sequenzspezifität (Komplementarität zu Zielsequenzen, Kodierung für trans-dominante Mutanten (Herskowitz, 1987)) eine intrazelluläre Immunität (Baltimore, 1988) gegen HIV bewirken und bei der Therapie des AIDS-Syndroms oder zur Verhinderung der Aktivierung des Virus nach der Infektion verwendet werden können.

Die pharmazeutischen Zubereitungen können z.B. verwendet werden, um im menschlichen oder tierischen Organismus virale Sequenzen, z.B. HIV oder verwandte Retroviren zu inhibieren.

Für die Anwendung der vorliegenden Erfindung kommt auch die Therapie nicht-viraler Leukämien in Betracht. Die Beteiligung von Onkogenen (abl, bcr, Ha, Ki, ras, rat, c-myc, N-myc) an der Entstehung lymphatischer Leukämien wurde in jüngster Zeit nachgewiesen; die Existenz weiterer Onkogene wird aufgrund von beobachteten spezifischen Chromosomentranslokationen für wahrscheinlich erachtet. Die Klonierung dieser Oncogene bietet die Grundlage für die Konstruktion oncogen-inhibierender Nukleinsäuremoleküle und damit für eine weitere therapeutische Einsatzmöglichkeit der vorliegenden Erfindung.

Ein weiteres wichtiges Anwendungsgebiet ist die Gentherapie. Prinzipiell können im Rahmen der Gentherapie mit Hilfe der vorliegenden Erfindung all diejenigen Gene bzw. Abschnitte davon in die Zielzellen eingeführt werden, deren Expression in diesem Zelltyp einen therapeutischen Effekt erzielt, z.B. durch Substitution genetisch bedingter Defekte oder Auslösen einer Immunantwort.

Für die therapeutische Anwendung kann die pharmazeutische Zubereitung systemisch, z.B. intravenös, verabreicht werden. Zielgewebe können die Lunge, die Milz, das Knochenmark und Tumore sein.

Beispiele für die lokale Anwendung sind die Lunge (Anwendung der erfindungsgemäßen pharmazeutischen Zubereitungen zur Instillation oder als Aerosol zur Inhalation), direkte Injektion ins Muskelgewebe, in einen Tumor oder in die Leber, oder die lokale Anwendung im Gastrointestinaltrakt oder in Blutgefäßabschnitten.

Die therapeutische Anwendung kann auch *ex vivo* durchgeführt werden, wobei die behandelten Zellen, z.B. Knochenmarkszellen oder Hepatozyten, in den Körper rückgeführt werden (z.B. Ponder et al., 1991).

### Figurenübersicht

- Fig.1:: Import von anti-CD4-Polylysin/pRSVL-Komplexen in CD4⁺-CHO-Zellen
- Fig.2:: Import von anti-CD4-Polylysin/pRSVL-Komplexen in CD4⁺-CHO-Zellen
- Fig.3, 4: Transfer und Expression von DNA in H9-Zellen mittels antiCD7-Polylysinl90-Konjugaten
- Fig.5:: Transfer von DNA in Pankreaskarzinomzellen mittels mAb1.1ASML-Polylysinl90-Konjugaten
- Fig.6:: Transfer von DNA in CD4⁺-Zellen mit Antikörper-Protein A-Polylysin-Konjugaten
- Fig.7:: Transfer von DNA in K562-Zellen mit Antikörper-Protein-A/G-Polylysin-Konjugaten

Die Erfindung wird anhand der nachfolgenden Beispiele illustriert.

### Vorversuch 1

### Herstellung von AntiCD4-Polylysin 90-Konjugaten

Die Kopplung erfolgte analog literaturbekannter Methoden durch Einführung von Disulfidbrücken nach Modifizierung mit Succinimidyl-Pyridyldithio-propionat (SPDP, Jung et al., 1981).

Eine Lösung von 1.7 mg antiCD4-Antikörper (OKT4A, Ortho Diagnostic Systems) in 50 mM Natriumphosphatpuffer pH 7.8 wurde mit 11 µl 10 mM ethanolischer Lösung von SPDP (Pharmacia) versetzt. Nach 1 h bei Raumtemperatur wurde über eine Gelsäule Sephadex G 25 filtriert (Eluens 100 mM HEPES-Puffer pH 7.3), wobei 1.4 mg antiCD4, modifiziert mit 75 nmol Pyridyldithiopropionatresten, erhalten wurden. Poly(L)lysin 90 (durchschnittlicher Polymerisationsgrad von 90 Lysinresten (Sigma), Fluoreszenzmarkierung mittels FITC) wurde analog mit SPDP modifiziert und durch Behandlung mit Dithiothreitol und nachfolgender Gelfiltration in die mit freien Mercaptogruppen modifizierte Form gebracht. Eine Lösung von 38 nmol Polylysin 90, modifiziert mit 120 nmol Mercaptogruppen, in 0.5 ml 20 mM Natriumacetatpuffer, wurde unter Sauerstoffausschluß mit oben angeführtem modifiziertem antiCD4 gemischt und über Nacht bei Raumtemperatur stehen gelassen. Die Isolierung der Konjugate erfolgte durch Gelpermeations-chromatographie (Superose 12, 500 mM Guanidinium-hydrochlorid, pH 7.3); nach Dialyse gegen 25 mM HEPES pH 7.3 wurden entsprechende Konjugate, bestehend aus 1.1 mg antiCD4-Antikörper, modifiziert mit 11 nmol Polylysin 90, erhalten.

### Vorversuch 2

### Herstellung von AntiCD4-Polylysin 190-Konjugaten

Eine Lösung von 1.0 mg (6.25nmol) antiCD4-Antikörper (OKT4A, Ortho Diagnostic Systems) in 0.3 ml 50 mM HEPES pH 7.8 wurde mit 37 µl 1 mM ethanolischer Lösung von Succinimidyl-Pyridyldithio-propionat (SPDP,Pharmacia) versetzt. Nach 1 h bei Raumtemperatur wurde über eine Gelsäule Sephadex G 25 filtriert (Eluens 100 mM HEPES-Puffer pH 7.9), wobei 0.85 mg (5.3 nmol) antiCD4, modifiziert mit 30 nmol Pyridyldithiopropionatresten, erhalten wurden. Poly(L)lysin190 (durchschnittlicher Polymerisationsgrad von 190 Lysinresten (Sigma), Fluoreszenzmarkierung mittels FITC) wurde analog mit SPDP modifiziert und durch Behandlung mit Dithiothreitol und nachfolgender Gelfiltration in die mit freien Mercaptogruppen modifizierte Form gebracht. Eine Lösung von 7.7 nmol Polylysinl90, modifiziert mit 25 nmol Mercaptogruppen, in 0.13 ml 30 mM Natriumacetatpuffer wurde unter Sauerstoffausschluß mit oben angeführtem modifiziertem antiCD4 (in 0.5 ml 300mM HEPES pH 7.9) gemischt und über Nacht bei Raumtemperatur stehen gelassen. Das Reaktionsgemisch wurde durch Zugabe von 5 M NaCl auf einen Gehalt von ungefähr 0.6 M gebracht. Die Isolierung der Konjugate erfolgte durch Ionenaustauschchromatographie (Mono S, Pharmacia, 50 mM HEPES pH 7.3, Salzgradient 0.6 M bis 3 M NaCl); nach Dialyse gegen 10 mM HEPES pH 7.3 wurden entsprechende Konjugate, bestehend aus 0.35 mg (2.2 nmol) antiCD4-Antikörper, modifiziert mit 3.9 nmol Polylysin190, erhalten.

### Vorversuch 3

### Herstellung von AntiCD7-Polylysin190-Konjugaten

Eine Lösung von 1.3 mg antiCD7-Antikörper (Immunotech) in 50 mM HEPES pH 7.9 wurde mit 49 µl 1 mM ethanolischer Lösung von SPDP (Pharmacia) versetzt. Nach 1 h bei Raumtemperatur wurde über eine Gelsäule Sephadex G 25 filtriert (Eluens 50 mM HEPES-Puffer pH 7.9) wobei 1.19 mg (7.5 nmol) antiCD7, modifiziert mit 33 nmol Pyridyldithiopropionatresten, erhalten wurden. Poly(L)lysinl90, Fluoreszenzmarkierung mittels FITC, wurde analog mit SPDP modifiziert und durch Behandlung mit Dithiothreitol und nachfolgender Gelfiltration in die mit freien Mercaptogruppen modifizierte Form gebracht.
Eine Lösung von 11 nmol Polylysinl90, modifiziert mit 35 nmol Mercaptogruppen, in 0.2 ml 30 mM Natriumacetatpuffer wurde unter Sauerstoffausschluß mit oben angeführtem modifiziertem antiCD7 (in 0.5 ml 300 mM HEPES pH 7.9) gemischt und über Nacht bei Raumtemperatur stehen gelassen. Das Reaktionsgemisch wurde durch Zugabe von 5 M NaCl auf einen Gehalt von ungefähr 0.6 M gebracht. Die Isolierung der Konjugate erfolgte durch Ionenaustauschchromatographie (Mono S, Pharmacia, 50 mM HEPES pH 7.3, Salzgradient 0.6 M bis 3 M NaCl); nach Dialyse gegen 10 mM HEPES pH 7.3 wurden entsprechende Konjugate, bestehend aus 0.51 mg (3.2 nmol) antiCD7-Antikörper, modifiziert mit 6.2 nmol Polylysinl90, erhalten.

### Beispiel 1

### Herstellung von mAbl.lASML-Polylysinl90-Konjugaten

In diesem Beispiel wurde ein monoklonaler Antikörper gegen die Membranproteinpräparation der Rattenpankreaskarzinom-Zellinie BSp73ASML (Matzku et al, 1983) mit der Bezeichnung mAbl.lASML für die Herstellung der Konjugate verwendet. Eine Lösung von 2.0 mg mAb1.1ASML in 0.5 ml 50 mM HEPES pH 7.9 wurde mit 75 µl 1 mM ethanolischer Lösung von SPDP (Pharmacia) versetzt. Nach 1 h bei Raumtemperatur wurde über eine Gelsäule Sephadex G 25 filtriert (Eluens 50 mM HEPES-Puffer pH 7.9), wobei 1.3 mg (8 nmol) mAbl.lASML, modifiziert mit 39 nmol Pyridyldithiopropionatresten, erhalten wurden. Poly(L)lysin190, Fluoreszenzmarkierung mittels FITC, wurde analog mit SPDP modifiziert und durch Behandlung mit Dithiothreitol und nachfolgender Gelfiltration in die mit freien Mercaptogruppen modifizierte Form gebracht.

Eine Lösung von 12 nmol Polylysin190, modifiziert mit 37 nmol Mercaptogruppen, in 210 µl 30 mM Natriumacetatpuffer wurde unter Sauerstoffausschluß mit oben angeführtem modifiziertem mAb1.1ASML (in 0.9 ml 100mM HEPES pH 7.9) gemischt und über Nacht bei Raumtemperatur stehen gelassen. Das Reaktionsgemisch wurde durch Zugabe von 5 M NaCl auf einen Gehalt von ungefähr 0.6 M gebracht. Die Isolierung der Konjugate erfolgte durch Ionenaustauschchromatographie (Mono S, Pharmacia, 50 mM HEPES pH 7.3, Salzgradient 0.6 M bis 3 M NaCl); nach Fraktionierung und Dialyse gegen 20 mM HEPES pH 7.3 wurden Konjugatfraktionen A, bestehend aus 0.16 mg (1 nmol) mAb1.1ASML, modifiziert mit 0.45 nmol Polylysinl90 (im Falle der Fraktion A), bzw. 0.23 mg (14 nmol) mAb1.1ASML, modifiziert mit 0.9 nmol Polylysinl90 (Fraktion B), bzw. 0.92 mg (5,8 nmol) mAb1.1ASML, modifiziert mit 3.9 nmol Polylysinl90 (Fraktion C) erhalten.

### Beispiel 2

### Herstellung von Protein-A-Polylysin190-Konjugaten

Eine Lösung von 4.5 mg Protein-A (Pierce, No. 21182, 107 nmol) in 0.5 ml 100 mM HEPES pH 7.9 wurde mit 30 µl 10 mM ethanolischer Lösung von SPDP (Pharmacia) versetzt. Nach 2 h bei Raumtemperatur wurde über eine Gelsäule Sephadex G 25 filtriert (Eluens 50 mM HEPES-Puffer pH 7.9) wobei 3.95 mg (94 nmol) Protein-A, modifiziert mit 245 nmol Pyridyldithiopropionatresten, erhalten wurden. Poly(L)lysin190, Fluoreszenzmarkierung mittels FITC, wurde analog mit SPDP modifiziert und durch Behandlung mit Dithiothreitol und nachfolgender Gelfiltration in die mit freien Mercaptogruppen modifizierte Form gebracht.
Eine Lösung von 53 nmol Polylysin190, modifiziert mit 150 nmol Mercaptogruppen, in 0.8 ml 30 mM Natriumacetatpuffer wurde unter Sauerstoffausschluß mit oben angeführtem modifiziertem Protein-A gemischt und über Nacht bei Raumtemperatur stehen gelassen. Das Reaktionsgemisch wurde durch Zugabe von 5 M NaCl auf einen Gehalt von ungefähr 0.6 M gebracht. Die Isolierung der Konjugate erfolgte durch Ionenaustauschchromatographie (Mono S, Pharmacia, 50 mM HEPES pH 7.3, Salzgradient 0.6 M bis 3 M NaCl); nach Fraktionierung und Dialyse gegen 25 mM HEPES pH 7.3 wurden zwei Konjugatfraktionen A und B, bestehend aus 1.15 mg (27 nmol) Protein-A, modifiziert mit 5 nmol Polylysinl90 (im Falle der Fraktion A) bzw. 2.6 mg (6.2 nmol) Protein-A, modifiziert mit 40 nmol Polylysin190 (Fraktion B), erhalten.

### Beispiel 3

### Herstellung von Komplexen von Antikörper-Polykation-Konjugaten mit DNA

Die Komplexe wurden hergestellt, indem verdünnte Lösungen von DNA (30 µg/ml oder weniger in 150 mM NaCl, 20 mM HEPES pH 7.3) mit den in den vorangegangenen Beispielen erhaltenen Antikörper-Polylysin-Konjugaten bzw. Protein A-Polylysin-Konjugaten (100 µg/ml oder weniger) vermischt wurden. Als DNA wurde pRSVL Plasmid-DNA (De Wet et al., 1987) verwendet, hergestellt mittels Triton-X Lyse Standard-Methode (Maniatis, 1982), gefolgt von CsCl/EtBr Gleichgewichtsdichtegradienten-Zentrifugation, Entfärbung mit Butanol-1 und Dialyse gegen 10 mM Tris/HCl pH 7.5, 1 mM EDTA.
Um ein Ausfällen der DNA-Komplexe zu verhindern, wurde phosphatfreier Puffer verwendet (Phosphate verringern die Löslichkeit der Konjugate).

### Vorversuch 4

### Transfer und Expression von DNA in CD4⁺ CHO-Zellen mittels antiCD4-Polylysin90-Konjugaten

In diesem und den folgenden Beispielen wurde Plasmid-DNA, enthaltend das Photinus pyralis-Luciferase-Gen als Reporter-Gen, verwendet, um Gentransfer und Expression zu untersuchen. In den Figuren, die die Ergebnisse der Versuche darstellen, beziehen sich die dargestellten Werte für die Luciferase-Aktivität auf die Aktivität der gesamten Zellprobe.

CD4⁺ CHO-Zellen (Lasky et al., 1987) wurden zu 5x10⁵ Zellen pro T-25-Flasche in Ham's F-12-Medium (Ham, 1965) plus 10 % FCS (fötales Kälberserum) ausgesät.
18 h später wurden die Zellen zweimal mit Ham's F-12-Medium ohne Serum gewaschen und in diesem Medium (5 ml) 5 h lang bei 37°C bebrütet.

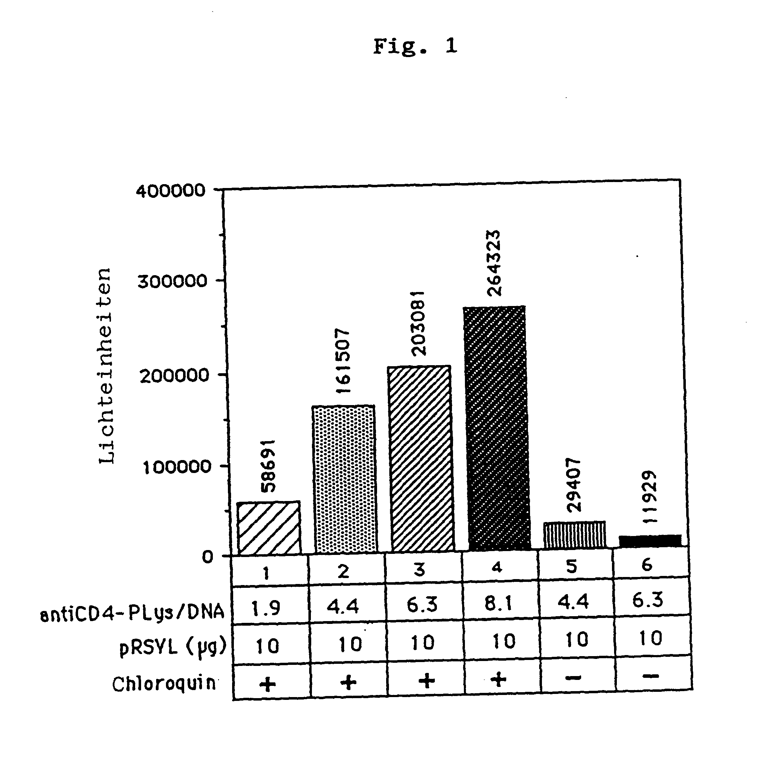

Anti-CD4-Polylysin/pRSVL Komplexe wurden bei Endkonzentrationen von DNA von 10 µg/500 µl in 150 mM NaCl, 20 mM HEPES pH 7.5 hergestellt, wie in Beispiel 6 beschrieben. Anti-CD4-Polylysin 90 (8.4 nmol Polylysin90/mg anti-CD4) wurde in den angegebenen Masse-Verhältnissen (von 1.9 bis 8.1, ausgedrückt als Masse von anti-CD4) eingesetzt. In den Proben 1 - 4 wurden die Komplexe den Zellen in Ham's F-12-Medium ohne Serum, enthaltend 100 µM Chloroquin zugefügt; in den Proben 5 und 6 wurde Chloroquin weggelassen. Nach einer 4stündigen Inkubation wurden die Zellen zweimal mit Medium plus 10 % FCS gewaschen und in diesem Medium inkubiert. In den Proben 5 und 6 wurde dasselbe Volumen an Serum enthaltendem Medium zu den Zellen gefügt. Nach 20 h wurden alle Zellen mit frischem, Serum enthaltendem Medium gewaschen und 48 h später geerntet. Aliquots von Extrakten (ca. 1/5 jeder Probe, entsprechend gleicher Proteinmenge, wurden auf Luciferase-Aktivität untersucht (De Wet et al., 1987). Die Bioluminiszenz wurde unter Verwendung des Clinilumats (Berthold, Wildbach, BRD) gemessen. Das Ergebnis dieser Untersuchungen ist in Fig.1 dargestellt, Es zeigte sich, daß DNA mit Hilfe der erfindungsgemäßen Konjugate in CD4⁺-Zellen importiert wird und die importierte DNA exprimiert wird, wobei die Effizienz des DNA-Imports proportional dem anti-CD4/Polylysin-Gehalt ist.

### Vorversuch 5

### Transfer und Expression von DNA in CD4⁺ CHO-Zellen mittels antiCD4-Polylysin190-Konjugaten

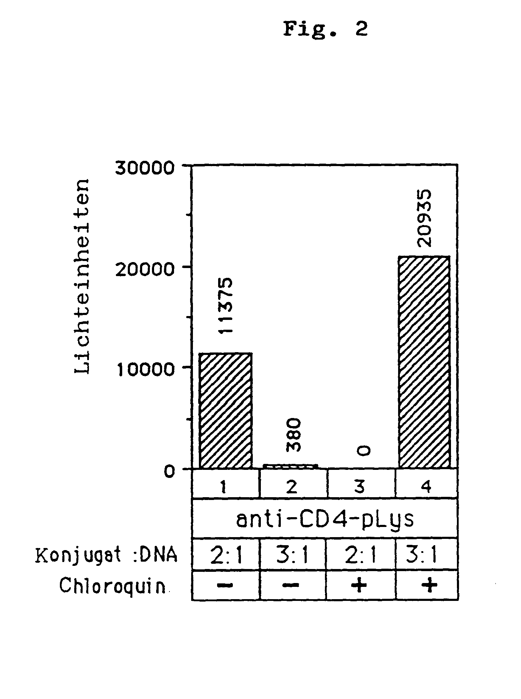

Zunächst wurden CD4⁺ CHO-Zellen gezüchtet, wie in Vorversuch 4 angegeben. Konjugat/DNA -Komplexe, hergestellt wie in Beispiel 3 angegeben, enthaltend 10 µg pRSVL und entweder einen 2:1 oder 3:1 Massenüberschuß an antiCD4-Polylysin 90 (s. Beispiel 1), wie in Fig.2 angegeben, wurden in Abwesenheit oder Gegenwart von 100 µM Chloroquin den Zellen hinzugefügt. Nach weiteren 4 h bei 37°C wurden die Proben mit Chloroquin zweimal mit Ham's Medium, enthaltend 10 % fötaleg Kälberserum gewaschen, während den Proben ohne Chloroquin 5 ml desselben Mediums zugefügt wurden. Die Zellen wurden weitere 20 h bei 37°C inkubiert und Aliquots auf Luciferaseaktivität untersucht, wie in Vorversuch 4 angegeben. Das Ergebnis dieser Versuche zeigt Fig.2.

### Vorversuch 6

### Transfer und Expression von DNA in H9-Zellen mittels antiCD7-Polylysin190-Konjugaten

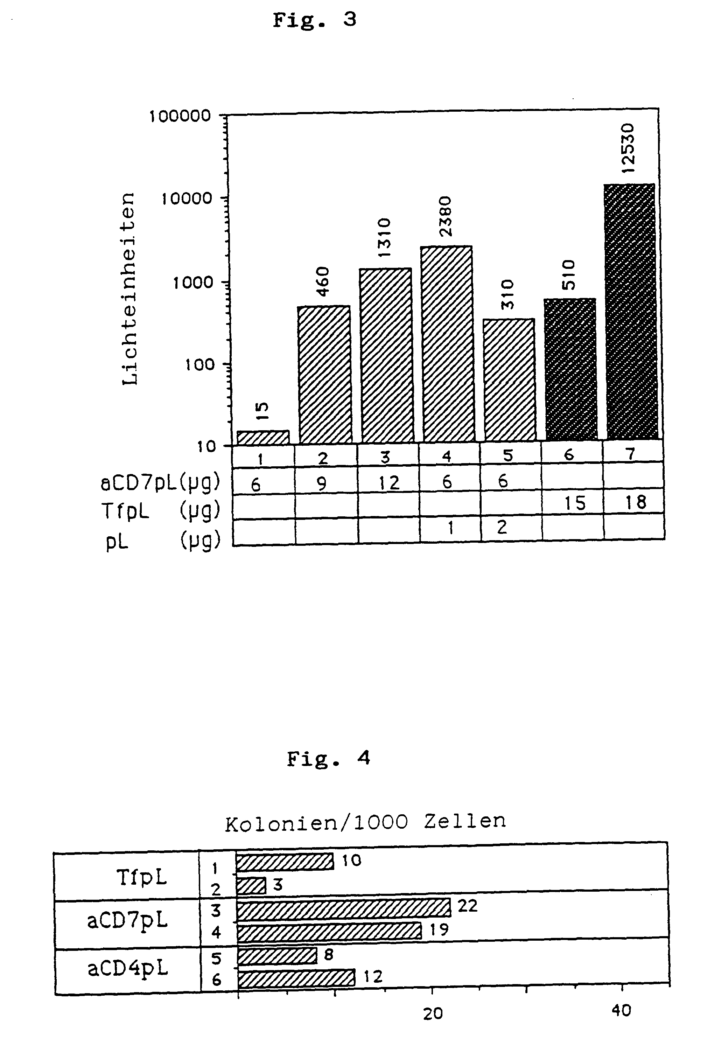

a) Zellen der T-Zellinie H9 (Mann et al., 1989) wurden in RPMI 1640 Medium, ergänzt mit 20 % FCS, 100 Einheiten/ml Penicillin, 100 µg/ml Streptomycin und 1 mM Glutamin gezüchtet. Unmittelbar vor der Transfektion wurden die Zellen durch Zentrifugation gesammelt und zu 100.000 Zellen/ml in frisches Medium aufgenommen (1,000.000 Zellen pro Probe) die zur Transfektion verwendet wurden. Zum Vergleich mit antiCD7-Konjugaten wurden Transferrin-Konjugate verwendet. Transferrin-Polylysin-Konjugate wurden hergestellt, wie in der EP-A 1 388 758 beschrieben; als antiCD7-Konjugate wurden die in Vorversuch 3 beschriebenen verwendet. Die Komplexierung mit DNA wurde vorgenommen, wie in Beispiel 3 angegeben. Für die transiente Transfektion in H9-Zellen wurde als DNA das Plasmid pHLuci verwendet, das die HIV-LTR-Sequenz, verbunden mit der für Luciferase kodierenden Sequenz, gefolgt von der SV40-Intron/polyA-Stelle, enthält: Das HindIII-Fragment, enthaltend das Protease 2A-Gen, von pHIV/2A (Sun und Baltimore, 1989) entfernt und durch ein HindIII/SmaI-Fragment von pRSVL (De Wet et al., 1987), enthaltend die für Luciferase kodierende Sequenz, ersetzt. Die beiden Fragmente wurden über die HindIII-Stellen (nach Herstellen glatter Enden mittels Klenow-Fragment) verbunden und dann über die glatte SMA1-Stelle mit der nunmehr glatten HindIII-Stelle verbunden. Ein Klon mit der richtigen Orientierung der Luciferase-Gensequenz wurde ausgewählt. Dieses Plasmid benötigt für starke Transkriptionsaktivität das TAT-Gen-Produkt. Dieses wird durch Co-Transfektion mit dem Plasmid pCMVTAT, welches für das HIV-TAT-Gen unter der Kontrolle des CMV immediate early promoter kodiert (Jakobovits et al., 1990) bereitgestellt. Die für die Transfektion verwendeten DNA-Komplexe enthielten eine Mischung von 5 µg pHLuci und 1 µg pCMVTAT. Die DNA/Polykation-Komplexe (500 µl) wurden zu der 10 ml Zellprobe hinzugegeben und 4 h lang in Gegenwart von 100 µM Chloroquin inkubiert. Anschließend wurden die Zellen in frisches Medium gewaschen, 40 h später geerntet und auf Luciferase-Aktivität untersucht, wie in den vorangegangenen Beispielen angegeben. Die Ergebnisse (in Luciferase-Lichteinheiten) sind in Fig. 3 dargestellt: Es zeigte sich, daß die Luciferase-Aktivität mit steigenden Mengen von antiCD7-Polylysin-Konjugat, komplexiert mit 6 µg DNA, zunimmt (Proben 1, 2 und 3). Eine weitere Steigerung der Aktivität wurde beobachtet, wenn 6 µg Konjugat zusammen mit 1 µg freiem Polylysin zur Komplexbildung verwendet wurden (Probe 4), ein weiterer Zusatz von Polylysin beeinträchtigte den Gen-Transport (Probe 5).(Die mit Transferrin-Polylysin-Konjugaten durchgeführten Vergleichsversuche sind mit 6 und 7 bezeichnet.)
b) Eine weitere Versuchsreihe zur Transfektion mit Hilfe der Antikörper-Konjugate wurde unter Verwendung des Plasmids pSSTNEO durchgeführt. Dieses Plasmid, das ein Neomycin-Resistenz-Gen als Marker enthält, wurde unter Verwendung von antiCD4-, antiCD7- und (zum Vergleich) Transferrin-Polylysin190-Konjugaten in H9-Zellen eingeführt (es wurden 6 µg DNA pro 10⁶ Zellen verwendet; die optimalen Transfektionsbedingungen waren in Vorversuchen mit Hilfe transienter Luciferase - Assays ermittelt worden). Das Plasmid pSSTNEO enthält das große Sst-Fragment des pUCu-Locus (Collis et al., 1990), der die HSV TK-neo-Einheit enthält. Ein 63 bp-Fragment, enthaltend eine einzige NdeI-Stelle war in die Asp718-Stelle eingeführt worden. Aliquots der transfizierten Zellen (enthaltend eine definierte Zellzahl) wurden daraufhin in ein halbfestes Methylcellulose-Medium, enthaltend 1000 µg/ml G418, verdünnt. Dazu wurden Aliquots der Zellen 3 Tage nach Transfektion mit DNA, enthaltend den Neomycin-Marker, in ein halbfestes Medium, das zusätzlich zu den normalen Anforderungen 0.5 - 1 mg/ml G418 und 20 mg/ml Methylcellulose enthielt, ausplattiert.(Zur Herstellung des halbfesten Selektionsmediums wurde unter sterilen Bedingungen eine Lösung von 20 g Methylcellulose in 460 ml Wasser hergestellt.) Daraufhin wurden 500 ml doppelt konzentriertes, ergänztes Nährmedium, ebenfalls hergestellt unter sterilen Bedingungen, mit der Methylcellulose-Lösung vereinigt, das Volumen auf 1 l gebracht und das Medium über Nacht bei 4°C gerührt. 50 ml Aliquots dieses Mediums wurden, gegebenenfalls nach Lagerung bei -20°C, mit je 10 ml Serum versetzt und das Volumen mit Komplettmedium ohne Serum auf 100 ml gebracht. In diesem Schritt wurde G418 hinzugefügt. Ein 2.5 ml Aliquot des Methylcellulose-Mediums wurde mit einem 50 bis 100 µl Aliquot der Zellsuspension vermischt und ca. 1 ml dieser Mischung in Kulturschalen ausgegossen. Die Bebrütung erfolgte bei 37°C unter CO₂-Atmosphäre. Ca. 10 bis 14 Tage später wurden die G418-resistenten Zellen gezählt (nur Kolonien mit mehr als 200 Zellen wurden als positiv gewertet). Die Ergebnisse sind in Fig.4 dargestellt (es ist die Zahl von G418 resistenten Kolonien/1000 Zellen 10 Tage nach Einbringen in das Antibiotika-Medium aufgetragen).

### Beispiel 4

### Transfer von DNA in Pankreaskarzinom-Zellen mittels mAb1.1ASML-Polylysin190-Konjugaten

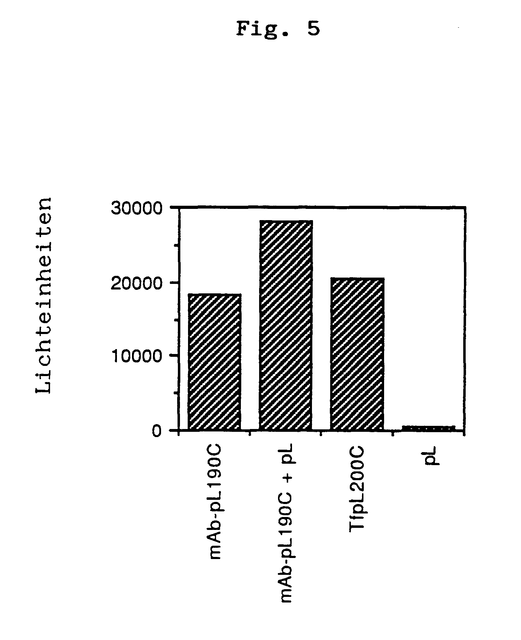

Zellen der metastasierenden Ratten-Pankreaskarzinom-Zellinie BSp73ASML (Matzku et al., 1989) in 2 ml RPMI 1640-Medium plus 10 % FCS wurden zu je 5 x 10⁵ Zellen in Platten (24-Wells, Falcon) angesetzt. Die Zellen wurden mit den in Beispiel 1 hergestellten mAb1,1ASML -Polylysinl90-Konjugaten (bzw. zum Vergleich mit Transferrin-Polylysin200-Konjugaten oder mit Polylysin allein), komplexiert mit pRSVL-DNA, zu den unten angegebenen Konjugat-DNA-Verhältnissen in Gegenwart von 100 µM Chloroquin in Berührung gebracht. Nach 4stündiger Inkubation bei 37°C mit den Komplexen wurde das Medium entfernt, frisches, Serum enthaltendes Medium (ohne Chloroquin) zugesetzt und die Zellen nach 20 h bei 37°C geerntet. Aus den Zellextrakten wurden Aliquots, standardisiert auf gleichen Proteingehalt, auf Luciferase-Aktivität untersucht. Die in Fig.5 angegebenen Werte für die Lichteinheiten entsprechen der Luciferase-Aktivität von 5 x 10⁵ Zellen, transfiziert mit 6 µg DNA (in der Figur bedeuten: mAb-pL190C: 18 µg mAb1.1ASML-pL190C-Konjugat; mAb-pL190C+pL: 9 µg mAb1.1ASML-pL190C-Konjugat + 1.5 µg nicht-konjugiertes Poly(L)lysin90; TfpL200: 18 µg TfpL200C (Transferrin-Polylysin200-Konjugat) und pL: 2.5 µg (bzw. 1 - 4 µg) Poly(L)lysin90).

### Beispiel 5

### Transfektion von CD4⁺ Zellen mit Antikörper-Protein A-Polylysin-Konjugaten

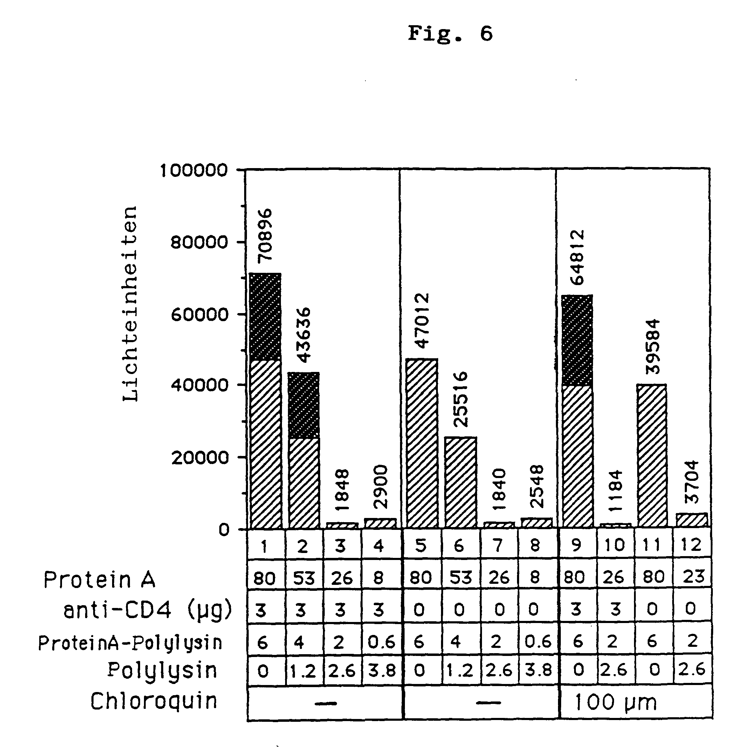

CD4-exprimierende HeLa-Zellen (s. Vorversuch 4) wurden zu 6 x 10⁵ Zellen/T25-Flasche ausgesät und anschließend in DME-Medium plus 10 % FCS gezüchtet. Wo in Fig. 6 angegeben, wurden die Zellen mit dem Antikörper (anti-CD4gp55kD, IOT4, Immunotech) (3 µg/Probe) 1 h lang bei Raumtemperatur vorinkubiert. In der Zwischenzeit wurden Protein A-Polylysin190/DNA-Komplexe in 500 µl HBS, enthaltend 6 µg pRSVL und die angegebenen Mengen Protein A-Polylysinl90 plus zusätzliches freies Polylysin, wie in Beispiel 6 hergestellt. Nach Ende der lstündigen Inkubation wurden die Zellen in 4.5 ml frisches Medium gegeben und die 500 µl DNA-Probe bei 37°C zu den Zellen zugegeben. Nach 4 h wurden diejenigen Proben, die 100 µM Chloroquin enthielten (Proben 9-12) in frisches Medium gewaschen, während die Proben 1-8 bis zur Ernte mit der DNA inkubiert wurden. Für den Luciferase-Assay wurden die Zellen 20 h später geerntet. Die Ergebnisse der Versuche sind in Fig. 6 dargestellt. Es zeigte sich, daß die Luciferase-Aktivität von der Gegenwart von Protein A-Polylysin im DNA-Komplex abhängig war (Proben 1-4, 5, 6). Bei den Proben 5-8, 11, 12 wurde DNA-Transport mittels des Protein A-Komplexes ohne Antikörper-Vorbehandlung nachgewiesen; der DNA-Import wurde jedoch um ca. 30 % gesteigert, wenn die Zellen mit dem Antikörper, der das Zelloberflächenprotein CD4 erkennt, vorbehandelt wurden (Proben 1-4, 9, 10). Es wurde weiters festgestellt, daß die Anwesenheit von Chloroquin keine Steigerung der DNA-Expression hervorruft (vgl. die Proben 1-8 mit den Proben 9-12).

### Beispiel 6

### Transfektion von K562-Zellen mit Antikörper-Protein-A/G-Polylysin 190-Konjugaten

a) Herstellung von Protein-A/G-Polylysin190-Konjugaten
   Eine Lösung von 4.5 mg rekombinantem Protein-A/G (Pierce, No. 21186, 102 nmol) in 0.5 ml 100 mM HEPES pH 7.9 wurde mit 30 µl 10 mM ethanolischer Lösung von SPDP (Pharmacia) versetzt. Nach 2 h bei Raumtemperatur wurde über eine Gelsäule Sephadex G 25 filtriert (Eluens 50 mM HEPES-Puffer pH 7.9), wobei 3.45 mg (75 nmol) Protein-A/G, modifiziert mit 290 nmol Pyridyldithiopropionatresten, erhalten wurden. Poly(L)lysin190, Fluoreszenzmarkierung mittels FITC, wurde analog mit SPDP modifiziert und durch Behandlung mit Dithiothreitol und nachfolgender Gelfiltration in die mit freien Mercaptogruppen modifizierte Form gebracht.
   Eine Lösung von 42 nmol Polylysin190, modifiziert mit 130 nmol Mercaptogruppen, in 0.8 ml 30 mM Natriumacetatpuffer wurde unter Sauerstoffausschluß mit oben angeführtem modifiziertem Protein-A/G gemischt und über Nacht bei Raumtemperatur stehen gelassen. Das Reaktionsgemisch wurde durch Zugabe von 5 M NaCl auf einen Gehalt von ungefähr 0.6 M gebracht. Die Isolierung der Konjugate erfolgte durch Ionenaustauschchromatographie (Mono S, Pharmacia, 50 mM HEPES pH 7.3, Salzgradient 0.6 M bis 3 M NaCl); nach Fraktionierung und Dialyse gegen 25 mM HEPES pH 7.3 wurde eine Konjugatfraktion, bestehend aus 1.02mg (22 nmol) Protein-A/G, modifiziert mit 12 nmol Polylysinl90 erhalten.
b) Herstellung von Antikörper-Protein-A/G-Polylysin190/DNA-Komplexen
   Eine Lösung von 7 µg des in a) hergestellten Protein-A/G-Konjugats in HBS wurde durch Mischen an den monoklonalen antiCD7-Antikörper, gerichtet gegen den Transferrinrezeptor, gebunden (3 µg, Klon BU55, IgG1, The Binding Site Limited, Birmingham, England). DNA-Komplexe wurden gebildet, indem eine Lösung des erhaltenen antiCD71-gebundenen Protein-A/G-Polylysin-Konjugats in 200 µl HBS mit einer Lösung von 6 µg Plasmid-DNA (enthaltend das Luciferasegen als Reportergen, vgl. die vorangegangenen Beispiele) in 300 µl HBS gemischt wurde.
c) Gentransfer in K562-Zellen

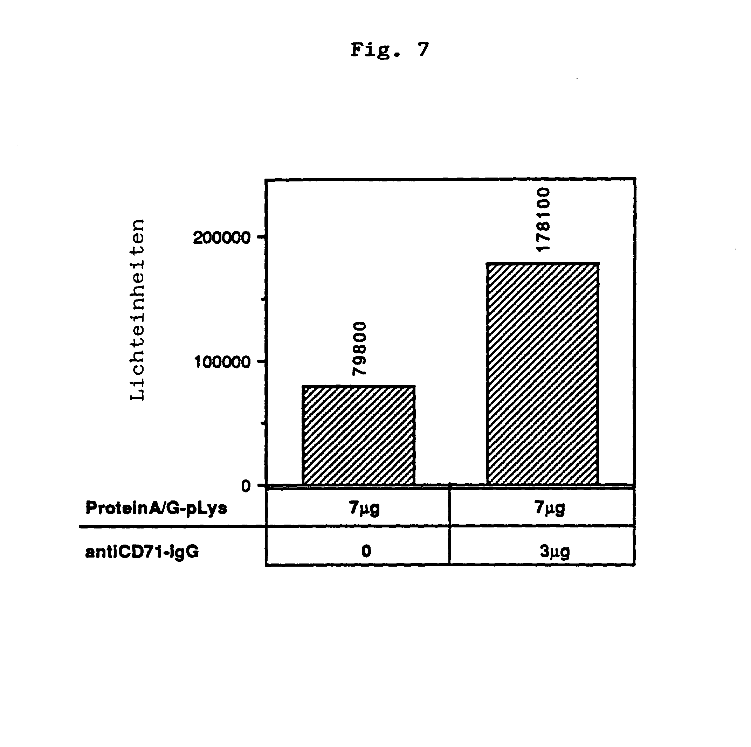

K562-Zellen (ATCC CCL243), die reich an Transferrinrezeptor sind, wurden in Suspension in RPMI 1640-Medium (Gibco BRL plus 2 g Natriumbicarbonat/l) plus 10 % FCS, 100 E/ml Penicillin, 100 µg/ml Streptomycin und 2 mM Glutamin, bis zu einer Dichte von 500.000 Zellen/ml gezüchtet. 20 h vor der Transfektion wurden die Zellen in frisches Medium, enthaltend 50 µM Deferrioxamin (Sigma), gegeben. Die Zellen wurden geerntet, in frischem Medium, enthaltend 10 % FCS (plus 50 µM Deferrioxamin), zu 250.000 Zellen/ml aufgenommen und in eine Platte mit 24 Vertiefungen gegeben (2 ml/Vertiefung). Während der ersten 4 h des Experiments enthielt das Medium 100 µM Chloroquin. Die Zellen wurden in frisches Medium ohne Chloroquin gewaschen und 24 h später geerntet. Die Luciferaseaktivität wurde bestimmt, wie in den vorangegangenen Beispielen angegeben. Das Ergebnis der Versuche ist in Fig. 7 dargestellt.

Literatur:
Aruffo A. und Seed B., 1987, EMBO J. 6, No. 11, 3313-3316
Baltimore D., 1988, Nature 335, 395
Chaudhary V.K. et al., 1990, Proc.Natl.Sci.USA 87, 1066
Ciliberto G. et al., 1985, Cell 41, 531-540
Co M.S. und Queen C., 1991, Nature 351, 501-502
Collis P. et al., 1990, EMBO J. 9, 233-240
De Wet et al., 1987, Mol.Cell.Biol. 7, 725-737
Green M. et al., 1989, 'Cell 58, 215-223
Ham R.G., 1965, Proc.Natl.Acad.Sci.USA 53, 288
Herskowitz I., 1987, Nature 329, 219
Jakobovits A. et al., 1990, EMBO J. 9, 1165-1170
Johnston S.A., 1990, Nature 346, 776-777
Jung et al., 1981, Biochem.Res.Commun. 101, 599
Kasid A. et al., 1990, Proc.Natl.Acad.Sci.USA 87, 473-477
Knapp W. et al., 1989, Immunol. Today 10, 253-258
Kurachi K. und Davie E.W., 1982, Proc.Natl.Acad.Sci.USA 79, 6461-6464
Lasky et al., 1987, Cell 50, 975-985
Luthman H.und Magnusson, 1983, Nucl.Acids Res.11 1295-1308
Malim M. et al., 1989, Cell 58, 205-214
Maniatis, 1982, Molecular Cloning, Cold Spring Harbor
Mann D.L. et al., 1989, AIDS Research and Human Retroviruses, 5, 253
Matzku S. et al., 1983, Invasion Metastasis 3, 109-123
Matzku S. et al., 1989, Cancer Res. 49, 1294-1299
Nabel E.G. et al., 1990, Science 249, 1285-1288
Orlandi R. et al., 1989, Proc.Natl.Acad.Sci.USA 86, 3833-3837
Pelchen-Matthews et al., 1989, EMBO J.8, 3641-3649
Ponder K.P. et al., 1991, Prod.Natl.Acad.Sci.USA 88, 1217-1221
Riordan J.R. et al., 1989, Science 245, 1066-1073
Sastry L. et al., 1989, Proc.Natl.Acad.Sci.USA 86, 5728-5732
Sun X.-H. und Baltimore D., 1989, Proc.Natl.Acad.Sci.USA 86, 2143-2146
Surolia A. et al., 1982, TIBS - February, 74-76
Trono D. et al., 1989, Cell 59, 113-120
Valerio D. et al., 1984, Gene 31, 147-153
Wagner E. et al., 1991, Bioconjugate Chemistry 2, 226-231
Waldmann T.A., 1991, Science 252, 1657-1662
Wilson J.M. et al., 1990, Proc.Natl.Acad.Sci.USA 87, 439-443
Wood W.I. et al., 1984, Nature 312, 330-337
Wu G.Y. und Wu C.H., 1987, J.Biol.Chem. 263, 14621-14624
Yang N.S. et al., 1990, Proc.Natl.Acad.Sci.USA 87, 9568-9572
Zamecnik et al., 1986, Proc.Natl.Acad.Sci.USA 83, 4143
Zon G., 1988, Pharmaceut. Research 5, No. 9, 539-549 Remington's Pharmaceutical Sciences, Mack Publishing Co., Easton, PA, Osol (ed.), 1980

## Patentansprüche

1. Protein-Polykation-Konjugate, die befähigt sind, mit Nukleinsäuren lösliche Komplexe zu bilden, dadurch gekennzeichnet, daß der Proteinanteil der Konjugate ein Antikörper bzw. ein Fragment davon, gerichtet gegen ein Zelloberflächenprotein der Zielzellen, mit der Fähigkeit ist, an das Zelloberflächenprotein zu binden, so daß die gebildeten Komplexe in menschliche oder tierische Zellen, die das Zelloberflächenprotein exprimieren, aufgenommen werden, ausgenommen ein Antikörper, gerichtet gegen ein T-Zell-Oberflächenprotein.

2. Konjugate nach Anspruch 1, dadurch gekennzeichnet, daß der Antikörper ein monoklonaler Antikörper bzw. ein Fragment davon ist.

3. Konjugate nach Anspruch 1 oder 2, dadurch gekennzeichnet, daß der Antikörper gegen ein Tumorantigen gerichtet ist.

4. Konjugate nach einem der Ansprüche 1 bis 3, dadurch gekennzeichnet, daß der Antikörper direkt an das Polykation gekoppelt ist.

5. Konjugate nach einem der Ansprüche 1 bis 3, dadurch gekennzeichnet, daß sie einen Antikörper enthalten, der über an Polykation gekoppeltes Protein A, das gegebenenfalls modifiziert ist, gebunden ist.

6. Protein A-Polykation-Konjugate zur Herstellung von Antikörper-Konjugaten nach Anspruch 5.

7. Konjugate nach nach einem der Ansprüche 1 bis 5, dadurch gekennzeichnet, daß das Polykation ein synthetisches homologes oder heterologes Polypeptid ist.

8. Konjugate nach Anspruch 7, dadurch gekennzeichnet, daß das Polypeptid Polylysin ist.

9. Konjugate nach einem der Ansprüche 1 bis 5, dadurch gekennzeichnet, daß das Polykation ein, gegebenenfalls modifiziertes, Protamin ist.

10. Konjugate nach nach einem der Ansprüche 1 bis 5, dadurch gekennzeichnet, daß das Polykation ein, gegebenenfalls modifiziertes, Histon ist.

11. Konjugate nach einem der Ansprüche 7 bis 10, dadurch gekennzeichnet, daß das Polykation ca. 20 bis 1000 positive Ladungen aufweist.

12. Konjugate nach einem der Ansprüche 7 bis 11, dadurch gekennzeichnet, daß das molare Verhältnis Antikörper:Polykation etwa 10:1 bis 1:10 beträgt.

13. Protein-Polykation/Nukleinsäure-Komplexe, die in menschliche oder tierische Zellen aufgenommen werden, dadurch gekennzeichnet, daß der Proteinanteil der Konjugate ein Antikörper, bzw. ein Fragment davon, gegen ein Zelloberflächenprotein der Zielzellen mit der Fähigkeit ist, an das Zelloberflächenprotein zu binden, so daß die gebildeten Komplexe in Zellen, die das Zelloberflächenprotein exprimieren, aufgenommen werden, ausgenommen ein Antikörper, gerichtet gegen ein T-Zell-Oberflächenprotein, wobei der Antikörper direkt oder über, gegebenenfalls modifiziertes, Protein A an das Polykation gebunden ist.

14. Komplexe nach Anspruch 13, dadurch gekennzeichnet, daß sie als Konjugat-Anteil eines der in den Ansprüchen 1 bis 5 oder 6 bis 12 definierten Konjugate enthalten.

15. Komplexe nach Anspruch 14, dadurch gekennzeichnet, daß sie zusätzlich ein nicht-kovalent gebundenes Polykation, das gegebenenfalls identisch ist mit dem Polykation des Konjugats, derart enthalten, daß die durch das Konjugat erzielte Internalisierung und/oder Expression der Nukleinsäure gesteigert wird.

16. Komplexe nach einem der Ansprüche 13 bis 15, dadurch gekennzeichnet, daß sie eine inhibierende Nukleinsäure in Form eines Antisense-Oligonukleotids oder Ribozyms bzw. des jeweils dafür kodierenden Gens, gegebenenfalls zusammen mit einem Carrier-Gen, enthalten.

17. Komplexe nach Anspruch 16, dadurch gekennzeichnet, daß die Nukleinsäure eine virusinhibierende Nukleinsäure ist.

18. Komplexe nach Anspruch 17, dadurch gekennzeichnet, daß sie eine Nukleinsäure enthalten, die Replikation und Expression des HIV-1 Virus oder verwandter Retroviren inhibiert.

19. Komplexe nach Anspruch 17 oder 18, dadurch gekennzeichnet, daß sie eine Nukleinsäure, kodierend für ein Virusprotein, das eine trans-dominante Mutation aufweist, enthalten.

20. Komplexe nach Anspruch 16, dadurch gekennzeichnet, daß sie eine Oncogen-inhibierende Nukleinsäure enthalten.

21. Komplexe nach einem der Ansprüche 13 bis 15, dadurch gekennzeichnet, daß sie eine therapeutisch oder gentherapeutisch wirksame Nukleinsäure in Form eines Gens oder eines Genabschnitts enthalten.

22. Verfahren zum Einführen von Nukleinsäure in höhere eukaryotische Zellen *in vitro* oder *ex vivo,* wobei man aus einem in einem der Ansprüche 1 bis 5 oder 7 bis 12 definierten Antikörper-Konjugate und Nukleinsäure(n), gegebenenfalls in Gegenwart von nicht-kovalent gebundenem Polykation, einen der in den Ansprüchen 13 bis 21 definierten, vorzugsweise unter physiologischen Bedingungen löslichen, Komplexe bildet und Zellen, die das Zelloberflächenprotein exprimieren, gegen das der Antikörper gerichtet ist, mit diesem Komplex, gegebenenfalls unter Bedingungen, unter denen der Abbau von Nukleinsäure in der Zelle inhibiert wird, in Berührung bringt.

23. Verfahren nach Anspruch 22 zum Einführen von Nukleinsäure in höhere eukaryotische Zellen, wobei man aus einem Protein A-Polykation-Konjugat, bestehend aus, gegebenenfalls modifiziertem, Protein A und einem der in den Ansprüchen 7 bis 11 definierten Polykationen sowie Nukleinsäure einen Komplex bildet und in Gegenwart eines Antikörpers, gerichtet gegen ein Zelloberflächenprotein der Zielzellen, mit Zellen, die dieses Zelloberflächenprotein exprimieren, in Berührung bringt, wobei der Antikörper an den Konjugat-Anteil des Komplexes gebunden wird.

24. Verfahren nach Anspruch 23, dadurch gekennzeichnet, daß die Zellen mit dem Antikörper vorbehandelt werden, bevor man sie mit dem Protein A-Polykation/Nukleinsäure-Komplex in Berührung bringt.

25. Pharmazeutische Zubereitung, enthaltend als wirksame Komponente eine oder mehrere therapeutisch oder gentherapeutisch wirksame Nukleinsäure(n) in Form eines der in den Ansprüchen 13 bis 21 definierten Komplexe.

## Claims

1. Protein-polycation conjugates which are capable of forming soluble complexes with nucleic acids, characterised in that the protein component of the conjugates is an antibody, or a fragment thereof, directed against a cell surface protein of the target cells, with the ability to bind to the cell surface protein, so that the complexes formed are absorbed into human or animal cells which express the cell surface protein, with the exception of an antibody directed against a T-cell surface protein.

2. Conjugates according to claim 1, characterised in that the antibody is a monoclonal antibody or a fragment thereof.

3. Conjugates according to claim 1 or 2, characterised in that the antibody is directed against a tumour antigen.

4. Conjugates according to one of claims 1 to 3, characterised in that the antibody is coupled directly to the polycation.

5. Conjugates according to one- of claims 1 to 3, characterised in that they contain an antibody which is bound via optionally modified protein A coupled to polycation.

6. Protein A-polycation conjugates for preparing antibody conjugates according to claim 5.

7. Conjugates according to one of claims 1 to 5, characterised in that the polycation is a synthetic homologous or heterologous polypeptide.

8. Conjugates according to claim 7, characterised in that the polypeptide is polylysine.

9. Conjugates according to one of claims 1 to 5, characterised in that the polycation is an optionally modified protamine.

10. Conjugates according to one of claims 1 to 5, characterised in that the polycation is an optionally modified histone.

11. Conjugates according to one of claims 7 to 10, characterised in that the polycation has about 20 to 1000 positive charges.

12. Conjugates according to one of claims 7 to 11, characterised in that the molar ratio of antibody to polycation is about 10:1 to 1:10.

13. Protein-polycation/nucleic acid complexes which are absorbed into human or animal cells, characterised in that the protein component of the conjugates is an antibody, or a fragment thereof, against a cell surface protein of the target cells, with the ability to bind to the cell surface protein, so that the complexes formed are absorbed into cells which express the cell surface protein, with the exception of an antibody directed against a T-cell surface protein, the antibody being bound directly to the polycation or via optionally modified protein A.

14. Complexes according to claim 13, characterised in that they contain as conjugate component one of the conjugates defined in claims 1 to 5 or 6 to 12.

15. Complexes according to claim 14, characterised in that they additionally contain a non-covalently bound polycation, which may optionally be identical to the polycation of the conjugate, so that the internalisation and/or expression of the nucleic acid achieved by means of the conjugate is increased.

16. Complexes according to one of claims 13 to 15, characterised in that they contain an inhibiting nucleic acid in the form of an antisense oligonucleotide or ribozyme or the gene coding therefor, optionally together with a carrier gene.

17. Complexes according to claim 16, characterised in that the nucleic acid is a virus-inhibiting nucleic acid.

18. Complexes according to claim 17, characterised in that they contain a nucleic acid which inhibits replication and expression of the HIV-1 virus or related retroviruses.

19. Complexes according to claim 17 or 18, characterised in that they contain a nucleic acid coding for a virus protein which has a transdominant mutation.

20. Complexes according to claim 16, characterised in that they contain an oncogene-inhibiting nucleic acid.

21. Complexes according to claim 13 to 15, characterised in that they contain a therapeutically or gene therapeutically active nucleic acid in the form of a gene or gene section.

22. Process for introducing nucleic acid into higher eukaryotic cells, in which one of the complexes defined in claims 13 to 21, preferably soluble under physiological conditions, is formed from an antibody conjugate as defined in one of claims 1 to 5 or 7 to 12 and nucleic acid or acids, optionally in the presence of non-covalently bound polycation, and cells which express the cell surface protein against which the antibody is directed are brought into contact with this complex, optionally under conditions in which the breakdown of nucleic acid in the cell is inhibited.

23. Process according to claim 22 for introducing nucleic acid into higher eukaryotic cells, in which a complex is formed from a protein A-polycation conjugate consisting of optionally modified protein A and one of the polycations defined in claims 7 toll and nucleic acid and in the presence of an antibody directed against a cell surface protein of the target cells the complex is brought into contact with cells which express this cell surface protein, the antibody being bound to the conjugate component of the complex.

24. Process according to claim 23, characterised in that the cells are pretreated with the antibody before being brought into contact with the protein A-polycation/nucleic acid complex.

25. Pharmaceutical preparation containing as active component one or more therapeutically or gene therapeutically active nucleic acids in the form of one of the complexes defined in claims 13 to 21.

## Revendications

1. Conjugués protéine-polycation, capables de former des complexes solubles avec des acides nucléiques, caractérisés en ce que l'élément protéinique des conjugués est un anticorps ou un de ses fragments dirigé contre une protéine de surface cellulaire des cellules cibles, qui a la capacité de se lier à la protéine de surface cellulaire, de façon que les complexes formés s'incorporent dans des cellules humaines ou animales qui expriment la protéine de surface cellulaire, à l'exception des anticorps dirigés contre une protéine de surface de lymphocytes T.

2. Conjugués selon la revendication 1, caractérisés en ce que l'anticorps est un anticorps monoclonal ou un de ses fragments.

3. Conjugués selon la revendication 1 ou 2, caractérisés en ce que l'anticorps est dirigé contre un antigène tumoral.

4. Conjugués selon l'une des revendications 1 à 3, caractérisés en ce que l'anticorps est couplé directement au polycation.

5. Conjugués selon l'une des revendications 1 à 3, caractérisés en ce qu'ils contiennent un anticorps qui est lié par l'intermédiaire de protéine A couplée au polycation et pouvant éventuellement être modifiée.

6. Conjugués protéine A ― polycation pour la préparation de conjugués d'anticorps selon la revendication 5.

7. Conjugués selon l'une des revendications 1 à 5, caractérisés en ce que le polycation est un polypeptide synthétique homologue ou hétérologue.

8. Conjugués selon la revendication 7, caractérisés en ce que le polypeptide est une polylysine.

9. Conjugués selon l'une des revendications 1 à 5, caractérisés en ce que le polycation est une protamine éventuellement modifiée.

10. Conjugués selon l'une des revendications 1 à 5, caractérisés en ce que le polycation est une histone éventuellement modifiée.

11. Conjugués selon l'une des revendications 7 à 10, caractérisés en ce que le polycation a environ 20 à 1 000 charges positives.

12. Conjugués selon l'une des revendications 7 à 11, caractérisés en ce que le rapport molaire anticorps:polycation est compris entre environ 10:1 et 1:10.

13. Complexes protéine-polycation/acide nucléique s'incorporant dans des cellules humaines ou animales, caractérisés en ce que l'élément protéinique des conjugués est un anticorps ou un de ses fragments dirigé contre une protéine de surface cellulaire des cellules cibles et ayant la capacité de se lier à la protéine de surface cellulaire de façon que les complexes formés s'incorporent dans des cellules qui expriment la protéine de surface cellulaire, à l'exception des anticorps dirigés contre une protéine de surface de lymphocytes T, l'anticorps étant lié au polycation directement ou par l'intermédiaire de protéine A éventuellement modifiée.

14. Complexes selon la revendication 13, caractérisés en ce qu'ils contiennent comme élément conjugué l'un des conjugués définis dans les revendications 1 à 5 ou 6 à 12.

15. Complexes selon la revendication 14, caractérisés en ce qu'ils contiennent en outre un polycation non lié par covalence, qui est éventuellement identique au polycation du conjugué, de façon à augmenter l'internalisation et/ou l'expression obtenue par le conjugué.

16. Complexes selon l'une des revendications 13 à 15, caractérisés en ce qu'ils contiennent un acide nucléique inhibiteur sous forme d'un oligonucléotide antisens ou d'un ribozyme ou du gène correspondant codant pour ces substances, éventuellement avec un gène support.

17. Complexes selon la revendication 16, caractérisés en ce que l'acide nucléique est un acide nucléique inhibiteur de virus.

18. Complexes selon la revendication 17, caractérisés en ce qu'ils contiennent un acide nucléique qui inhibe la réplication et l'expression du virus VIH-1 ou de rétrovirus apparentés.

19. Complexes selon la revendication 17 ou 18, caractérisés en ce qu'ils contiennent un acide nucléique codant pour une protéine virale qui présente une mutation trans-dominante.

20. Complexes selon la revendication 16, caractérisés en ce qu'ils contiennent un acide nucléique inhibiteur d'un oncogène.

21. Complexes selon l'une des revendications 13 à 15, caractérisés en ce qu'ils contiennent un acide nucléique ayant une activité en thérapeutique ou en thérapie génique, sous forme d'un gène ou d'un fragment de gène.

22. Procédé d'introduction d'acides nucléiques dans des cellules eucaryotes supérieures *in vitro* ou *ex vivo*, dans lequel on forme l'un des complexes définis dans les revendications 13 à 21, de préférence solubles dans des conditions physiologiques, à partir d'un conjugué d'anticorps défini dans l'une des revendications 1 à 5 ou 7 à 12 et d'un ou plusieurs acides nucléiques, éventuellement en présence d'un polycation non lié par covalence, et on met en contact des cellules, qui expriment la protéine de surface cellulaire contre laquelle est dirigé l'anticorps, avec ce complexe, éventuellement dans des conditions dans lesquelles la dégradation de l'acide nucléique dans la cellule est inhibée.

23. Procédé selon la revendication 22 pour l'introduction d'acides nucléiques dans des cellules eucaryotes supérieures, dans lequel on forme un complexe à partir d'un conjugué protéine A ― polycation constitué de protéine A éventuellement modifiée et de l'un des polycations définis dans les revendications 7 à 11, et d'un acide nucléique, et on le met en contact, en présence d'un anticorps dirigé contre une protéine de surface cellulaire des cellules cibles, avec des cellules qui expriment cette protéine de surface cellulaire, l'anticorps se liant à l'élément conjugué du complexe.

24. Procédé selon la revendication 23, caractérisé en ce que l'on soumet les cellules à un traitement préalable avec l'anticorps avant de les mettre en contact avec le complexe protéine A - polycation/acide nucléique.

25. Composition pharmaceutique contenant comme constituant actif un ou plusieurs acides nucléiques actifs en thérapeutique ou en thérapie génique, sous forme de l'un des complexes définis dans les revendications 13 à 21.
